# EUROPEAN PATENT APPLICATION

(11) **EP 1 837 400 A1**
(43) Date of publication of application: **26.09.2007**
(21) Application number: 05822820.6
(22) Date of filing: 27.12.2005
(51) Int. Cl.: C12N 15/55, C07K 16/40, C12N 1/15, C12N 1/19, C12N 1/21, C12N 5/10, C12N 9/16, C12P 21/08, C12Q 1/04, C12Q 1/34, C12Q 1/68, G01N 33/569, G01N 33/574

(54) **NOVEL TANNASE GENE AND PROTEIN THEREOF**

(30) Priority: 05.01.2005 JP 2005000845
(71) Applicant: Eisai R&D Management Co., Ltd., Tokyo 112-8088 (JP)
(72) Inventor: SASATSU, Masanori, Hino-shi, Tokyo 1910024 (JP); NOGUCHI, Masahisa, Hachioji-shi, Tokyo 1920363 (JP); MORIYASU, Fuminori, Tokyo 1040051 (JP)
(74) Representative: Woods, Geoffrey Corlett
(86) International application number: PCT/JP2005/024247
(87) International publication number: WO 2006/073133

(57) **Abstract**

A polynucleotide encoding a protein as defined in the following (a), (b) or (c):
(a) a protein comprising the amino acid sequence as shown in SEQ ID NO: 2;
(b) a protein comprising the amino acid sequence as shown in SEQ ID NO: 2 with one or several amino acids substituted, deleted, inserted or added, and having a tannase activity;
(c) a protein comprising an amino acid sequence showing 90% or more homology to the amino acid sequence as shown in SEQ ID NO: 2, and having a tannase activity.

SEQUENCE LISTING

<110> Eisai R&D Managemant Co., Ltd.

<120> Tannase gene and protein thereof

<130> G07-0037

<150> JP2005-00845
<151> 2005-01-05

<160> 15

<170> PatentIn version 3.2

<210> 1
<211> 1842
<212> DNA
<213> Staphylococcus lugdunensis

<220>
<221> CDS
<222> (1)..(1839)

<400> 1
atg aaa aag act ttc ata tca ctc tta tcc gca aca gtt ata ctt tca 48
Met Lys Lys Thr Phe Ile Ser Leu Leu Ser Ala Thr Val Ile Leu Ser
1               5                   10                  15

ggt tgt ggc gtt ggc gaa cat caa aat aat aat tct aat cat gat gct 96
Gly Cys Gly Val Gly Glu His Gln Asn Asn Asn Ser Asn His Asp Ala
            20                  25                  30

aaa ggt gtg aac act tca aat gtt aaa atc aaa aat tat aac caa gca 144
Lys Gly Val Asn Thr Ser Asn Val Lys Ile Lys Asn Tyr Asn Gln Ala
        35                  40                  45

tca tct gcg ctg caa ata gat aat tca aaa tgg aaa tat gat agt aaa 192
Ser Ser Ala Leu Gln Ile Asp Asn Ser Lys Trp Lys Tyr Asp Ser Lys
    50                  55                  60

aat aac gtt tat tat caa cta aat ata agt tat gtc tcc aat ccc caa 240
Asn Asn Val Tyr Tyr Gln Leu Asn Ile Ser Tyr Val Ser Asn Pro Gln
65                  70                  75                  80

gct aaa aat gta gaa aaa tta ggt atc tat gta cca gct gct tat ttc 288
Ala Lys Asn Val Glu Lys Leu Gly Ile Tyr Val Pro Ala Ala Tyr Phe
                85                  90                  95

aaa ggt aaa aag aat cat aat ggg aca tat acc gtt act gta aac gat 336
Lys Gly Lys Lys Asn His Asn Gly Thr Tyr Thr Val Thr Val Asn Asp
            100                 105                 110

gct aag aaa gtt aac ggc tat tct gct aga aca gca cct atc gtt tat 384
Ala Lys Lys Val Asn Gly Tyr Ser Ala Arg Thr Ala Pro Ile Val Tyr
        115                 120                 125

cca gtc aat aca cct ggt tat gcc gaa caa agt gca cct acg tca tat 432
Pro Val Asn Thr Pro Gly Tyr Ala Glu Gln Ser Ala Pro Thr Ser Tyr
    130                 135                 140

cgt tat agt aat att tct aag tat atg aaa gct gga ttc ata tat gtt 480
Arg Tyr Ser Asn Ile Ser Lys Tyr Met Lys Ala Gly Phe Ile Tyr Val
145                 150                 155                 160

gaa gca gga tta cga gga cgt agt atg agc atg ggc aat aac agc agt 528
Glu Ala Gly Leu Arg Gly Arg Ser Met Ser Met Gly Asn Asn Ser Ser
                165                 170                 175

aat gca tca act aaa tca tat gaa acc ggt tct cct tgg ggt gta acc 576
Asn Ala Ser Thr Lys Ser Tyr Glu Thr Gly Ser Pro Trp Gly Val Thr
            180                 185                 190

gat ctt aaa gca gca atc aga tat tac cgt ttc aac gat agt agt cta 624
Asp Leu Lys Ala Ala Ile Arg Tyr Tyr Arg Phe Asn Asp Ser Ser Leu

        195                 200                 205

cca ggt aac agt agt aag att tat act ttt ggt cat agt ggc ggt ggt 672
Pro Gly Asn Ser Ser Lys Ile Tyr Thr Phe Gly His Ser Gly Gly Gly
    210                 215                 220

gct caa agt gct att gcc ggt gca tca ggt gat agc aag ctc tac tat 720
Ala Gln Ser Ala Ile Ala Gly Ala Ser Gly Asp Ser Lys Leu Tyr Tyr
225                 230                 235                 240

aaa tat tta gaa caa att ggc gca gcc atg aca gat aaa aat gga aaa 768
Lys Tyr Leu Glu Gln Ile Gly Ala Ala Met Thr Asp Lys Asn Gly Lys
                245                 250                 255

tat atc agt gat aaa att gac ggt gct atg gcg tgg tgc cct att aca 816
Tyr Ile Ser Asp Lys Ile Asp Gly Ala Met Ala Trp Cys Pro Ile Thr
            260                 265                 270

agt cta gat caa gcc gat gct gct tat gaa tgg caa atg gga caa tat 864
Ser Leu Asp Gln Ala Asp Ala Ala Tyr Glu Trp Gln Met Gly Gln Tyr
        275                 280                 285

ggt aat gaa ggt aat cgc aag aaa aat tca ttc caa aaa caa tta tca 912
Gly Asn Glu Gly Asn Arg Lys Lys Asn Ser Phe Gln Lys Gln Leu Ser
    290                 295                 300

acc gat tta gca tca tct tat gca agc tac tta aat aaa cta aat ctg 960
Thr Asp Leu Ala Ser Ser Tyr Ala Ser Tyr Leu Asn Lys Leu Asn Leu
305                 310                 315                 320

aaa aat gga aat act aca tta tca tta act aaa tct aaa aat ggt caa 1008
Lys Asn Gly Asn Thr Thr Leu Ser Leu Thr Lys Ser Lys Asn Gly Gln
                325                 330                 335

tat act gaa ggc tca tat gct aaa tat cta aaa aaa gaa att gaa gat 1056
Tyr Thr Glu Gly Ser Tyr Ala Lys Tyr Leu Lys Lys Glu Ile Glu Asp
            340                 345                 350

tca gct aca gaa ttc tta aat aat aca aca ttc cct tac aaa caa aat 1104
Ser Ala Thr Glu Phe Leu Asn Asn Thr Thr Phe Pro Tyr Lys Gln Asn
        355                 360                 365

agc act gag caa gca ggc atg ggt aat ggt gga cct agc ggt gga aaa 1152
Ser Thr Glu Gln Ala Gly Met Gly Asn Gly Gly Pro Ser Gly Gly Lys
    370                 375                 380

cct tct ggc aaa atg gga tct atg cct caa atg aga aaa caa tct tca 1200
Pro Ser Gly Lys Met Gly Ser Met Pro Gln Met Arg Lys Gln Ser Ser
385                 390                 395                 400

aat aaa aca tac aaa aca atg gat gct tac tta aaa gat cta aat aaa 1248
Asn Lys Thr Tyr Lys Thr Met Asp Ala Tyr Leu Lys Asp Leu Asn Lys
                405                 410                 415

aaa ggc aca tgg atc acg tat gat aag aaa aca aaa cgc gca cat att 1296
Lys Gly Thr Trp Ile Thr Tyr Asp Lys Lys Thr Lys Arg Ala His Ile
            420                 425                 430

aca agt ctt aaa gac ttt gcg aaa tat tat aaa caa cct tct aaa tca 1344
Thr Ser Leu Lys Asp Phe Ala Lys Tyr Tyr Lys Gln Pro Ser Lys Ser
        435                 440                 445

gtt tca gcc ttt gat gat tta aaa cgt agc caa gct gaa aat gaa gtg 1392
Val Ser Ala Phe Asp Asp Leu Lys Arg Ser Gln Ala Glu Asn Glu Val
    450                 455                 460

ttt gga aca tca ggt agt gac agt aaa tta cat ttt gat caa tca cta 1440
Phe Gly Thr Ser Gly Ser Asp Ser Lys Leu His Phe Asp Gln Ser Leu
465                 470                 475                 480

gct aaa ctt tta aca gaa aat aaa tct aac tat agc aaa cta aat ggt 1488
Ala Lys Leu Leu Thr Glu Asn Lys Ser Asn Tyr Ser Lys Leu Asn Gly
                485                 490                 495

tgg aat agt aac tat gtt tca tca tat aaa aat gac tta aca aaa aca 1536
Trp Asn Ser Asn Tyr Val Ser Ser Tyr Lys Asn Asp Leu Thr Lys Thr

500                 505                 510

gat aaa tta ggc aca agc atg tca aca aga atg aat atg tac aat cca        1584
Asp Lys Leu Gly Thr Ser Met Ser Thr Arg Met Asn Met Tyr Asn Pro
        515                 520                 525

atg tat tac tta tct gat tac tat agc ggg tat ggt aaa tct aat gtg        1632
Met Tyr Tyr Leu Ser Asp Tyr Tyr Ser Gly Tyr Gly Lys Ser Asn Val
    530                 535                 540

gca aat cat tgg aga att aga aca ggt att caa caa gga gat acg gcc        1680
Ala Asn His Trp Arg Ile Arg Thr Gly Ile Gln Gln Gly Asp Thr Ala
545                 550                 555                 560

tta aat act gaa act aat ctt tcg cta gct tta aaa gaa cgc gtt ggt        1728
Leu Asn Thr Glu Thr Asn Leu Ser Leu Ala Leu Lys Glu Arg Val Gly
                565                 570                 575

tct aaa aac gtt gac ttc aaa aca gtt tgg gat caa ggt cat aca atg        1776
Ser Lys Asn Val Asp Phe Lys Thr Val Trp Asp Gln Gly His Thr Met
            580                 585                 590

gca gaa aca tca ggt aat agt gat agt aac ttc atc aaa tgg gta gaa        1824
Ala Glu Thr Ser Gly Asn Ser Asp Ser Asn Phe Ile Lys Trp Val Glu
        595                 600                 605

agt att aat aaa aaa tag                                                1842
Ser Ile Asn Lys Lys
    610

<210>  2
<211>  613
<212>  PRT
<213>  Staphylococcus lugdunensis

<400>  2

Met Lys Lys Thr Phe Ile Ser Leu Leu Ser Ala Thr Val Ile Leu Ser
1               5                   10                  15

Gly Cys Gly Val Gly Glu His Gln Asn Asn Asn Ser Asn His Asp Ala
            20                  25                  30

Lys Gly Val Asn Thr Ser Asn Val Lys Ile Lys Asn Tyr Asn Gln Ala
        35                  40                  45

Ser Ser Ala Leu Gln Ile Asp Asn Ser Lys Trp Lys Tyr Asp Ser Lys
    50                  55                  60

Asn Asn Val Tyr Tyr Gln Leu Asn Ile Ser Tyr Val Ser Asn Pro Gln
65                  70                  75                  80

Ala Lys Asn Val Glu Lys Leu Gly Ile Tyr Val Pro Ala Ala Tyr Phe
                85                  90                  95

Lys Gly Lys Lys Asn His Asn Gly Thr Tyr Thr Val Thr Val Asn Asp
            100                 105                 110

Ala Lys Lys Val Asn Gly Tyr Ser Ala Arg Thr Ala Pro Ile Val Tyr
        115                 120                 125

Pro Val Asn Thr Pro Gly Tyr Ala Glu Gln Ser Ala Pro Thr Ser Tyr
    130                 135                 140

Arg Tyr Ser Asn Ile Ser Lys Tyr Met Lys Ala Gly Phe Ile Tyr Val
145                 150                 155                 160

Glu Ala Gly Leu Arg Gly Arg Ser Met Ser Met Gly Asn Asn Ser Ser
                165                 170                 175

Asn Ala Ser Thr Lys Ser Tyr Glu Thr Gly Ser Pro Trp Gly Val Thr
            180                 185                 190

Asp Leu Lys Ala Ala Ile Arg Tyr Tyr Arg Phe Asn Asp Ser Ser Leu
        195                 200                 205

Pro Gly Asn Ser Ser Lys Ile Tyr Thr Phe Gly His Ser Gly Gly Gly
    210                 215                 220

Ala Gln Ser Ala Ile Ala Gly Ala Ser Gly Asp Ser Lys Leu Tyr Tyr
225                 230                 235                 240

Lys Tyr Leu Glu Gln Ile Gly Ala Ala Met Thr Asp Lys Asn Gly Lys
                245                 250                 255

Tyr Ile Ser Asp Lys Ile Asp Gly Ala Met Ala Trp Cys Pro Ile Thr
            260                 265                 270

Ser Leu Asp Gln Ala Asp Ala Ala Tyr Glu Trp Gln Met Gly Gln Tyr
        275                 280                 285

Gly Asn Glu Gly Asn Arg Lys Lys Asn Ser Phe Gln Lys Gln Leu Ser
    290                 295                 300

Thr Asp Leu Ala Ser Ser Tyr Ala Ser Tyr Leu Asn Lys Leu Asn Leu
305                 310                 315                 320

Lys Asn Gly Asn Thr Thr Leu Ser Leu Thr Lys Ser Lys Asn Gly Gln
                325                 330                 335

Tyr Thr Glu Gly Ser Tyr Ala Lys Tyr Leu Lys Lys Glu Ile Glu Asp
            340                 345                 350

Ser Ala Thr Glu Phe Leu Asn Asn Thr Thr Phe Pro Tyr Lys Gln Asn
        355                 360                 365

Ser Thr Glu Gln Ala Gly Met Gly Asn Gly Gly Pro Ser Gly Gly Lys
    370                 375                 380

Pro Ser Gly Lys Met Gly Ser Met Pro Gln Met Arg Lys Gln Ser Ser
385                 390                 395                 400

Asn Lys Thr Tyr Lys Thr Met Asp Ala Tyr Leu Lys Asp Leu Asn Lys
                405                 410                 415

Lys Gly Thr Trp Ile Thr Tyr Asp Lys Lys Thr Lys Arg Ala His Ile
            420                 425                 430

Thr Ser Leu Lys Asp Phe Ala Lys Tyr Tyr Lys Gln Pro Ser Lys Ser
        435                 440                 445

Val Ser Ala Phe Asp Asp Leu Lys Arg Ser Gln Ala Glu Asn Glu Val
    450                 455                 460

Phe Gly Thr Ser Gly Ser Asp Ser Lys Leu His Phe Asp Gln Ser Leu
465                 470                 475                 480

Ala Lys Leu Leu Thr Glu Asn Lys Ser Asn Tyr Ser Lys Leu Asn Gly
                485                 490                 495

Trp Asn Ser Asn Tyr Val Ser Ser Tyr Lys Asn Asp Leu Thr Lys Thr
            500                 505                 510

Asp Lys Leu Gly Thr Ser Met Ser Thr Arg Met Asn Met Tyr Asn Pro
        515                 520                 525

Met Tyr Tyr Leu Ser Asp Tyr Tyr Ser Gly Tyr Gly Lys Ser Asn Val
    530                 535                 540

Ala Asn His Trp Arg Ile Arg Thr Gly Ile Gln Gln Gly Asp Thr Ala
545                 550                 555                 560

Leu Asn Thr Glu Thr Asn Leu Ser Leu Ala Leu Lys Glu Arg Val Gly
                565                 570                 575

Ser Lys Asn Val Asp Phe Lys Thr Val Trp Asp Gln Gly His Thr Met
            580                 585                 590

Ala Glu Thr Ser Gly Asn Ser Asp Ser Asn Phe Ile Lys Trp Val Glu
        595                 600                 605

Ser Ile Asn Lys Lys
    610

<210>  3
<211>  22
<212>  DNA
<213>  Artificial

<220>
<223>  primer

<400>  3
gatgtgctgc aaggcgaatt gt                                              22

<210>  4
<211>  24
<212>  DNA
<213>  Artificial

<220>
<223>  primer

<400>  4
gctcgtatgt tgtgtggaat tgtg                                            24

<210>  5
<211>  6994
<212>  DNA
<213>  Staphylococcus lugdunensis

<400>  5
aagcttgagc aacttctttg tccaatgtgg taactaatgc tttattgaaa taagaagttt      60

caatgcaaag cggcttatcg tctaaataac gaatgcgctc tactaaatag acttgtgtat     120

ctttatcttt tatctgtaag tgtttttgtg tttcctcgtt aggcggtatg atgtcgatgt 180
taagcacttt acttgttaca cgatgtcctt gtaaattatc tgaaaagccc tttgttttta 240
acagattaat atatccagat tttttgctat ttctaacata tataccactg ccccgagttt 300
gatagataat accatctttt tcaagtaact ctaatgcttt aacaatcgta cttttactca 360
cttgatacgc tatttttagt tctgctaggc ttggaagttt gtcaccaact ttatatttcc 420
catttgcaat agctgcggta attccatgtg caacatgttc gtactttaac atgattaatc 480
tccttaatgc tgtatttaca atatagtata ccaaaaataa gctgaaatta aattgtaccg 540
gtataattgt ataaaaacga tttacaaatt gtaccggtat aattataatg agttgtgtaa 600
gggtttccat atttgggata aataaaaatt aaatagattg ggaggttgtg ttatgagtga 660
aagagataga aaattagcga tacatatcct tgaagccgtt ggtggaaagg aaaatatcgt 720
taattacacg cattgcgcaa cgcgattacg tatcgtctta aaacgttcta ttccggatgc 780
taaaaagaaa gtagcaagta tggatggcgt agtaacggta gttgaaaata atggacaatt 840
tcaagtggtt attggcaatg cggtcgcaag tgtttatcaa gaatttgagg gattattggg 900
agaagaaaat caacaatcta gcgatgaaga cagtgaaaaa ggtacgatta ttaatcgtat 960
tattgcaacg atgtctgccg tctttgcacc atttatttat gtcttagcgg ctgccggtat 1020
attacaaggt atattaatta ttgcgaaaat gttgttttca tcatttgaga agactggcac 1080
atatgaagta tttaacttta tttcatgggc accatttaca tttttaccaa tatttattgc 1140
gattactgca gcaaaacatt tcaaagtgaa tacttatatc gcagtagctt gttgtgctgc 1200
actcgttagc ccggatttag caggaattgt agaacgcatt gggagtggac atacgtttac 1260
atttattggt attccattaa gtgaaacaag ctatacatca tcagtgttac caccactatt 1320
attagtatgg gtgttgtcat atgttgaaaa atatttaaat aaatatattc atgaagttat 1380
acgcccgtta tttgtaccgt tcttcagtat tgtaattatg gtgccattaa cattattaat 1440
tattggtcca ttagcatctg gggctgcaca tggtattgca caaggcttta actggctcgt 1500
agctgtagca ccatggttag caggattatt aattggtgca ttttggcagg tatttgttat 1560
ctttggtgtt cattggggca ttacgccaat ggtattggca aactttgaac agtataaaag 1620
tgatgctttc caagcctttc aaacaattgc tgttattgct caatttggtg cagttatggg 1680
tgtattcttc aaaacgagaa ataaggattt aaagcgtgta tcgttatcag cagggattac 1740
aggcatcttt ggaattactg aaccgtctat gtatggtgtg aatttgcgct ttaaaaagcc 1800
attcattatc gcttgtatta gtggtggtat tggggcttta gttgcaagtt tctttaatcc 1860
aaaatattat gcatatgctg gtcttcctgg accgataaca attgtcaatg gttatagtcc 1920
tgataacatc tcatcaatat ggggaatttt acttggttca gccgttgcaa ttattttacc 1980
tatcatttta atccaagtac ttggctatgg tgaagatgtg acagaacagg tagaacgtgt 2040
tgacgtcgaa gaaggacatg agataaatga acaaacacgt ttgactacta catctgatca 2100
acttgaaaca gagatacatg ctccactaaa aggtttagta gtagcattat ctgaagtaga 2160
tgatccaata tttgcggaag gtatgatggg taaaggatta gccattcagc cagaaagtta 2220
tgaagtgaca gcacctctca aaggggtgat tagtatggtt gcagtaagta aacacgcgat 2280
aggtattaca tcgccagagg ggatcgaact tttagttcac gttggtttag atactgtgca 2340
attaaatgga caaggctttg aattacttgt tgctgaaaat gatcaggtcg aagaaggcca 2400

gcctttgctg agatttgatg caaattatat taaagaacag ggctacgaac ttattacacc 2460
tgtaattatt actaacagtg atgaatttga agacattatc atagctgaca gtcaaaatat 2520
cacatctaga gaaaaaataa tgacagtaat taaaaaatag agtgtaaagg agatttaaac 2580
atggctaaat taccacaaga tttcttatgg ggcggcgcac ttgctgccaa tcaatttgaa 2640
ggtggctatg accaaggtgg caaaggctta agtgtaattg atgtaatgac agcaggcgca 2700
catggtcaag cacgtgaaat tacacaagat atagaacctg ataaatatta tcctaatcat 2760
atgggtattg atttttacca tcgctataaa gaagatgtcg cattgtttgc tgaaatggga 2820
ttgaaatgtt tgcgtacatc gattgcatgg acacgtattt tccctaatgg taatgaaaca 2880
gaaccgaacg aagcaggact gcaattctat gatgatttat ttgatgaatt attgaaatat 2940
gacattcaac cagtgattac attatcgcat tttgagatgc cactacatct agcacgtgaa 3000
tatggcggtt ttagaaaccg taaagtagca gattattttg catattttgc tgaaactgta 3060
tttaagcgtt ataaagacaa agttaaatac tggatgacat ttaatgaaat taataaccaa 3120
atggacatca ataatcctat tttcttatgg acaaactctg gtgtatcgtt gacagatgat 3180
gataataaat tagaagtatt atatcaggtg gcacataatg aattgattgc aagtgcgaaa 3240
gcagtaaaaa ttggtaagga gataaatcca gattttgaaa ttggtgctat gatttcccat 3300
gttccaatct acccatatag ctgtaatccg caagatataa tggaggcaga aatcgcgagt 3360
cgtatgcgtt tcttctttcc agatgtacaa gcacgaggtt attacccagg ttatgcgaca 3420
aaaatgtttg aacgtgaagg cttcgacatt ggttggcaag aaggagacga tgtgattcta 3480
cgtgaaggta aagtcgatta tattggattt agttattata tgtctacggt cgttaaatct 3540
gataatgagg ctagtgtaga agataatata gtaaatggtg gcttacccaa ttcggttgat 3600
aatccttata ttaagaaaag tgattggggc tgggccattg atccggttgg cttacgttat 3660
acattaaatg cattatataa tcgttatcaa attcctttat ttatagttga aaatggattt 3720
ggtgccgtag atgagtttga tgataaaggt aaaatccatg acgaatatcg tatcgagtat 3780
ttacgtgaac atattgaagc ggctattgat gcagtagatc aagatggcgt agaattaatg 3840
ggctatacac cttggggtat tattgatatt gtatccttta cgacaggtga aatgaaaaaa 3900
cgttatggtt taattcatgt tgatcgtgat aatgaaggaa acggttcatt agaacgtacc 3960
aagaaagatt catttgactg gtatcgcaaa gttattgagt caaatgggga agttttgtaa 4020
attaaaaaga gagctgaaca gaattcatta tgaattctta gtccagctct ggcaaggttg 4080
actaagattg aaaaagcatg ctataagcat attttcaatt gagtcaacta ctgccaatca 4140
ttagttcaca gcctaaaaca tgatgatgtt ttaggctgtt taatacattt agctattttt 4200
tattaatact ttctacccat ttgatgaagt tactatcact attacctgat gtttctgcca 4260
ttgtatgacc ttgatcccaa actgttttga agtcaacgtt tttagaacca acgcgttctt 4320
ttaaagctag cgaaagatta gtttcagtat ttaaggccgt atctccttgt tgaatacctg 4380
ttctaattct ccaatgattt gccacattag atttaccata cccgctatag taatcagata 4440
agtaatacat tggattgtac atattcattc ttgttgacat gcttgtgcct aatttatctg 4500
tttttgttaa gtcattttta tatgatgaaa catagttact attccaacca tttagtttgc 4560
tatagttaga tttattttct gttaaaagtt tagctagtga ttgatcaaaa tgtaatttac 4620
tgtcactacc tgatgttcca aacacttcat tttcagcttg gctacgtttt aaatcatcaa 4680

aggctgaaac tgatttagaa ggttgtttat aatatttcgc aaagtcttta agacttgtaa 4740
tatgtgcgcg ttttgttttc ttatcatacg tgatccatgt gcctttttta tttagatctt 4800
ttaagtaagc atccattgtt ttgtatgttt tatttgaaga ttgttttctc atttgaggca 4860
tagatcccat tttgccagaa ggttttccac cgctaggtcc accattaccc atgcctgctt 4920
gctcagtgct attttgtttg taagggaatg ttgtattatt taagaattct gtagctgaat 4980
cttcaatttc tttttttaga tatttagcat atgagccttc agtatattga ccatttttag 5040
atttagttaa tgataatgta gtatttccat ttttcagatt tagtttattt aagtagcttg 5100
cataagatga tgctaaatcg gttgataatt gtttttggaa tgaatttttc ttgcgattac 5160
cttcattacc atattgtccc atttgccatt cataagcagc atcggcttga tctagacttg 5220
taatagggca ccacgccata gcaccgtcaa ttttatcact gatatatttt ccatttttat 5280
ctgtcatggc tgcgccaatt tgttctaaat atttatagta gagcttgcta tcacctgatg 5340
caccggcaat agcactttga gcaccaccgc cactatgacc aaaagtataa atcttactac 5400
tgttacctgg tagactacta tcgttgaaac ggtaatatct gattgctgct ttaagatcgg 5460
ttacacccca aggagaaccg gtttcatatg atttagttga tgcattactg ctgttattgc 5520
ccatgctcat actacgtcct cgtaatcctg cttcaacata tatgaatcca gctttcatat 5580
acttagaaat attactataa cgatatgacg taggtgcact ttgttcggca taaccaggtg 5640
tattgactgg ataaacgata ggtgctgttc tagcagaata gccgttaact ttcttagcat 5700
cgtttacagt aacggtatat gtcccattat gattcttttt acctttgaaa taagcagctg 5760
gtacatagat acctaatttt tctacatttt tagcttgggg attggagaca taacttatat 5820
ttagttgata ataaacgtta tttttactat catatttcca ttttgaatta tctatttgca 5880
gcgcagatga tgcttggtta taatttttga ttttaacatt tgaagtgttc acacctttag 5940
catcatgatt agaattatta ttttgatgtt cgccaacgcc acaacctgaa agtataactg 6000
ttgcggataa gagtgatatg aaagtctttt tcatctaatt agcctcctta atttgtcgta 6060
gttctattat tagtcgcaaa taaggagcat atcaattcgg attgagcgca tgaaccattc 6120
taaattagaa agatgatgta caccttaggt ctcttgacag tatttgcttg aaattagttt 6180
ctgtgctttt tggctaactc tagcaaatat gccacctctt tagaagcaat taaatgatta 6240
tgaacattaa cgttaatata gagttttata ggtgaattca gctgaacgat ttttaaagga 6300
gacgttacgt aataattagc ctgttgttcc ataatcaatg cgacgccatt atttgtattt 6360
accatttcaa gtaaattttc aatacgaggt gctgtaaatg aaattaatgg ataaaattgt 6420
tgttcttcac aataatttaa aaaagcttga tgctccaatg cgaactcagg tggtaaatag 6480
agttgttcat tttcaagcat cgatagatcg atttgtgaga cataggctaa tggatgagat 6540
tcatgcacaa tagcacataa gctttcttca atataagtaa aagaatgatt atcatgaggt 6600
ggatgtggtg atcttaaaaa gctaatgtca atcttacctt cattaatcaa ttgagctaat 6660
tcttgttctg acccttcatg aatacgaaat ctaatattag aaaatttttg aacacatttt 6720
gagattaagt ttgtaatacc atattcaatc attaatcgag aagcgccaag gttaatggtg 6780
attaaattat cattttcctg ttgctcaagc tgttttgata aatctttata attttggcaa 6840
atgattttgg cataatgcag aaatagttta cctgattctg ttagttcaaa ttgtttattt 6900
gcgcgatcta taagacgata accgcactca ttttccaaag aaacaatgtg cttagacagt 6960

gtagattgcg ctatgtagag ttcttgagaa gctt                                    6994

<210>  6
<211>  29
<212>  DNA
<213>  Artificial

<220>
<223>  primer

<400>  6
gaaagaatcc aatatttgcg gaaggtatg                                          29

<210>  7
<211>  27
<212>  DNA
<213>  Artificial

<220>
<223>  primer

<400>  7
gaaagtcgac ttcaaaacag tttggga                                            27

<210>  8
<211>  29
<212>  DNA
<213>  Artificial

<220>
<223>  primer

<400>  8
gaaagtcgac tggtatcgca aagttattg                                          29

<210>  9
<211>  29
<212>  DNA
<213>  Artificial

<220>
<223>  primer

<400>  9
gaaaggatcc acctcatgat aatcattct                                          29

<210>  10
<211>  36
<212>  DNA
<213>  Artificial

<220>
<223>  primer

<400>  10
gaaaggatcc atgaaaaaga ctttcatatc actctt                                  36

<210>  11
<211>  38
<212>  DNA
<213>  Artificial

<220>
<223>  primer

<400>  11
ggaaaactcg agctattttt tattaatact ttctaccc                                38

<210>  12

<211>  23
<212>  DNA
<213>  Artificial

<220>
<223>  primer

<400>  12
gggctggcaa gccacgtttg gtg                                               23


<210>  13
<211>  23
<212>  DNA
<213>  Artificial

<220>
<223>  primer

<400>  13
ccgggagctg catgtgtcag agg                                               23


<210>  14
<211>  20
<212>  DNA
<213>  Artificial

<220>
<223>  primer

<400>  14
cgtggtgccc tattacaagt                                                   20


<210>  15
<211>  22
<212>  DNA
<213>  Artificial

<220>
<223>  primer

<400>  15
tgagccttca gtatattgac ca                                                22

## Description

### TECHNICAL FIELD

The present invention relates to a tannase gene possessed by a tannase-positive bacterium in feces, a tannase protein encoded by the gene, a method of diagnosing colon cancer by detecting the gene or the protein, and a diagnostic reagent for colon cancer.

### BACKGROUND ART

*Streptococcus (St.) bovis* is known as a pathogenic bacterium of infectious endocarditis, and high concurrent incidence of infectious endocarditis due to this bacterium and colon cancer attracts attention in Europe and United States (Honberg P. Z. et al., Lancet, i: 163-164, 1987). Meanwhile, Osawa et al. isolated a bacterium having a tannase activity, which hydrolyzes an ester bond in tannic acid to release gallic acid, from feces of koala eating eucalyptus containing tannin at a high concentration, and identified this bacterium as *St. bovis* biotype I (Osawa R. et al., Appl. Environ. Microbiol., 56: 829-831, 1999). This bacterium has the tannase activity for degrading tannin to release gallic acid, and contains decarboxylase that decarboxylates gallic acid into pyrogallol, and it is proposed that this bacterium should be newly designated as *St. gallolyticus*. Osawa inferred a possibility that this *St. gallolyticus* is a bacterium identical to *St. bovis* isolated from those colon cancer patients concurrently having infectious endocarditis (Osawa R., RIKEN Symposium Abstracts, "Classification and Ecology of Lactic Acid Bacteria", pp. 36-45, 1996).

In general, the substance referred to as tannin belongs to polyphenols, natural substances constituting an important portion of phenolic compounds, and has toxic and growth-inhibitory actions on microorganisms as well as an astringent taste. While various classifications of tannin have been proposed, tannin is largely classified into hydrolysable tannin and condensed tannin for convenience. The former is structurally pyrogallol tannin, and it is also referred to as pathological tannin since it is contained in nutgall or gall nut in a large amount. On the other hand, the latter is catechol tannin, and it is also referred to as physiological tannin since it is a normal component of plants. The hydrolysable tannin has a chemical structure in which phenolic acid (gallic acid, ellagic acid, etc.) binds to a saccharide as a core through an ester bond. It is known that this hydrolysable tannin is hydrolyzed by tannase, which is a tannin-degrading enzyme mainly produced by fungi such as *Aspergillus* and *Candida* living in soil. However, production of tannase by bacteria living in the intestinal canal of animals had not been reported before the publication of Osawa.

Since then, it has been reported that *Lonepinella koalarum* was isolated from feces of koala as a tannase-positive bacterium, in addition to *St. gallolyticus*, and also isolated mainly from herbivorous animals (Osawa R., Syst. Appl. Microbiol., 15: 144-147, 1992). Recently, it has been further reported that *Lactobacillus (L.) plantarum* was isolated from human feces. *L. plantarum* is a lactobacillus mainly isolated from silos, and infectious endocarditis caused by *L. plantarum* has also been reported (Oakey H. J. et al., J. Appl. Bacteriol., 78: 142-148, 1995).

With respect to the involvement of tannase-producing bacteria in human colon cancer, it has been reported that tannase-producing bacteria are found in feces of a large number of colon cancer patients (WO 02/34938) and that isolation rates of tannase-producing bacteria from feces are high in colon cancer patients among those patients who underwent endoscopy of the lower gastrointestinal tract (Hagiwara T. et al., the 90th Meeting of the Japanese Society of Gastroenterology, Abstract A225, 2004). Hagiwara et al. isolated tannase-producing bacteria using a tannin-treated agar medium and reported that tannase-positive bacteria were detected in 10.6% of healthy subjects, 33.3% of patients with benign polyps, 25.0% of patients with early colon cancer, and 54.5% of patients with advanced colon cancer. These results suggest the relation between colon cancer and tannase-producing bacteria.

WO 02/34938 also reports that the tannase activity of *Staphylococcus (S.) lugdunensis* is especially strong among tannase-producing bacteria and that a large number of the tannase-producing bacteria isolated from colon cancer patients are *S. lugdunensis.*

Tannase-producing bacteria are isolated as follows. Briefly, bacteria are cultured in a tannic acid-treated agar medium under anaerobic conditions at 37 °C for 7 days. Then, colonies surrounded by a clear zone formed by the degradation of tannin are selected, and their tannase activities are measured according to the method of Osawa et al. (Osawa R. et al., Appl. Environ. Microbiol., 59, 1251-1252, 1993). Since this method is complicated in operation and requires more than one week until the results are obtained, a simple and prompt method for detecting tannase-producing bacteria applicable to general clinical tests is demanded.

### DISCLOSURE OF THE INVENTION

It is an object of the present invention to develop a simple gene detection method or immunochemical detection method for detecting a tannase-producing bacterium which can be an indicator for diagnosing colon cancer.

Under circumstances, the present inventors have isolated from feces of colon cancer patients *S*. *lugdunensis* (a bacterium with a relatively strong tannase activity), cloned a tannase gene from this bacterium and determined the nucleotide sequence of the gene. The thus cloned tannase gene was a novel gene completely different from the already reported, *Aspergillus*-derived tannase gene (Hatamoto O. et al., Gene, 175, 215-221, 1996).

Then, the present inventors have attempted to detect tannase genes in bacteria cultured from human feces by PCR, and found that a tannase-producing bacterium is detected in a sample from which a tannase gene has been detected. Thus, the present inventors have achieved a simple method for detecting a tannase-producing bacterium.

The present invention relates to the following.
(1) A polynucleotide encoding a protein as defined in the following (a), (b) or (c):
   (a) a protein comprising the amino acid sequence as shown in SEQ ID NO: 2;
   (b) a protein comprising the amino acid sequence as shown in SEQ ID NO: 2 with one or several amino acids substituted, deleted, inserted or added, and having a tannase activity;
   (c) a protein comprising an amino acid sequence showing 90% or more homology to the amino acid sequence as shown in SEQ ID NO: 2, and having a tannase activity.
(2) A polynucleotide as defined in the following (a) or (b):
   (a) a polynucleotide comprising the coding region of the polynucleotide sequence as shown in SEQ ID NO: 1;
   (b) a polynucleotide which hybridizes to a DNA consisting of a nucleotide sequence complementary to a DNA comprising the nucleotide sequence as shown in SEQ ID NO: 1 under stringent conditions, and which encodes a protein having a tannase activity.
(3) A protein as defined in the following (a), (b) or (c):
   (a) a protein comprising the amino acid sequence as shown in SEQ ID NO: 2;
   (b) a protein comprising the amino acid sequence as shown in SEQ ID NO: 2 with one or several amino acids substituted, deleted, inserted or added, and having a tannase activity;
   (c) a protein comprising an amino acid sequence showing 90% or more homology to the amino acid sequence as shown in SEQ ID NO: 2, and having a tannase activity.
(4) A vector comprising the polynucleotide according to (1) or (2) above.
(5) A host cell comprising the polynucleotide according to (1) or (2) above or the vector according to (4) above.
(6) A method of producing the protein according to (3) above, comprising culturing the host cell according to (5) above and collecting the protein from the host cell or culture supernatant thereof
(7) A polynucleotide for use in detecting *Staphylococcus lugdunensis,* which hybridizes to the polynucleotide according to (1) or (2) above under stringent conditions and has a strand length of at least 15 nucleotides.
(8) An antibody or a fragment thereof which binds to the protein according to (3) above.
(9) The antibody or fragment thereof according to (8) above, wherein the antibody is a monoclonal antibody.
(10) A monoclonal antibody or a fragment thereof which binds to tannase, wherein said monoclonal antibody is produced by a hybridoma obtained by cell fusion between a myeloma cell and an antibody producing cell from an animal immunized with the protein according to (3) above or a part thereof
(11) A hybridoma which is obtained by cell fusion between a myeloma cell and an antibody producing cell from an animal immunized with the protein according to (3) above or a part thereof, and produces a monoclonal antibody that binds to tannase.
(12) A method of producing the monoclonal antibody, comprising culturing the hybridoma according to (11) above and collecting from the resultant culture a monoclonal antibody that binds to tannase.
(13) A method of detecting *Staphylococcus lugdunensis* by contacting the polynucleotide according to (7) above with a test sample.
(14) A method of detecting or diagnosing colon cancer, comprising detecting *Staphylococcus lugdunensis* by contacting the polynucleotide according to (7) above with.a test sample.
(15) A method of detecting tannase-producing *Staphylococcus lugdunensis* by contacting the antibody or fragment thereof according to any one of (8) to (10) above with a test sample.
(16) A method of detecting or diagnosing colon cancer, comprising detecting a tannase-positive bacterium by contacting the antibody or fragment thereof according to any one of (8) to (10) above with a test sample.
(17) A diagnostic reagent for colon cancer, comprising the polynucleotide according to (7) above.
(18) A diagnostic reagent for colon cancer, comprising the antibody or fragment thereof according to any one of (8) to (10) above.
(19) A kit for detecting *Staphylococcus lugdunensis* or colon cancer, comprising at least one selected from the group consisting of the polynucleotide according to (7) above and the antibody or fragment thereof according to (8) to (10) above.

### BRIEF DESCRIPTION OF THE DRAWINGS

Fig. 1 is a physical map of a DNA fragment comprising a tannase gene.
Fig. 2 is a diagram showing the results of SDS polyacrylamide gel electrophoresis of a GST-fused tannase protein.
Fig. 3 is a diagram showing the results of confirmation of antibody specificity by Western blotting.
Fig. 4 is a graph showing the results of GST tannase measurement by EIA.
Fig. 5 is a graph showing the results of *S. lugdunensis* tannase measurement by EIA.

### BEST MODE FOR CARRYING OUT THE INVENTION

Hereinbelow, embodiments of the present invention, i.e., the method of cloning and identification of a tannase gene, the method of preparation of a tannase protein expression vector, the method of preparation of antibody to the tannase protein, and the method of detection of tannase will be described in detail.

The following embodiments are provided for the purpose of illustrating the present invention, and it is not intended to limit the present invention to these embodiments. The present invention may be practiced in many ways without deviation from the spirit of the present invention.

All publications, patents, patent applications and other patent documents cited herein are incorporated herein by reference in their entirety

### <Outline of the Invention>

The present invention relates to an *S. lugdunensis*-derived tannase gene, a tannase protein encoded by the gene, an antibody to the tannase protein, and diagnosis of colon cancer by detecting a tannase-producing bacterium using the gene and the antibody.

Tannase is an enzyme catalyzing a reaction which degrades tannin (a natural substance constituting an important portion of the compounds called polyphenols) to release gallic acid. Tannin is classified into hydrolysable tannin (the so-called pathological tannin) and condensed tannin which is catechol tannin and is also called physiological tannin since it is a normal component of plants. It is hydrolysable tannin that tannase degrades.

Tannase-positive bacteria which produce this tannase are mainly isolated from herbivorous animals, and some are detected in human feces. It is known that tannase-producing bacteria are isolated at high rates especially from feces of colon cancer patients. Then, it is contemplated that a tannase gene or an antibody to tannase may be supplied to a simple colon cancer diagnosis as an indicator.

### <Tannase>

The tannase in the present invention (hereinafter, sometimes referred to as the "protein of the present invention") encompasses a protein comprising the amino acid sequence as shown in SEQ ID NO: 2, preferably a protein consisting of the amino acid sequence as shown in SEQ ID NO: 2, and a protein consisting of an amino acid sequence substantially identical to the amino acid sequence as shown in SEQ ID NO: 2.

Hereinbelow, the protein of the present invention will be described in detail.

Examples of amino acid sequences substantially identical to the amino acid sequence as shown in SEQ ID NO: 2 include, for example, amino acid sequences which have about 90% or more, preferably about 95% or more, more preferably about 98% or more homology to the amino acid sequence as shown in SEQ ID NO: 2 and have a tannase activity. Homology may be determined using homology search sites in the internet [e.g., homology search such as FASTA, BLAST, PSI-BLAST and SSEARCH may be used at Japan DNA Databank (DDBJ); see, for example, Search and Analysis page in DDBJ website: http://www.ddbj.nig.ac.jp/E-mail/homology-j.html; further, search using BLAST may be performed at National Center for Biotechnology Information (NCBI) (for example, go to the BLAST page of NCBI website: http://www.ncbi.nlm.nih.gov/BLAST/; Altschul, S.F. et al., J. Mol. Biol., 1990, 215(3):403-10; Altschul, S.F. & Gish, W., Meth. Enzymol., 1996, 266:460-480; Altschul, S.F. et al., Nucleic Acids Res., 1997, 25:3389-3402)].

In addition to the above-described amino acid sequences, examples of amino acid sequences substantially identical to the amino acid sequence as shown in SEQ ID NO: 2 include the amino acid sequence as shown in SEQ ID NO: 2 which has a mutation such as deletion, insertion, substitution or addition or a combination of these mutations in one or more (e.g., one or several) amino acids and encodes a protein having a tannase activity. The expression "having a tannase activity" means to have 10% or more, 30% or more, preferably 90% or more activity compared to the tannase activity of the protein consisting of the amino acid sequence as shown in SEQ ID NO: 2. Tannase activity means an activity that functions to degrade hydrolysable tannin to release gallic acid. This activity may be measured by the method described in WO 02/34938.

For example, (i) the amino acid sequence as shown in SEQ ID NO: 2 with deletion of 1 to 5 (preferably 1 to 3, more preferably 1 to 2, still more preferably 1) amino acids; (ii) the amino acid sequence as shown in SEQ ID NO: 2 with addition of 1 to 5 (preferably 1 to 3, more preferably 1 to 2, still more preferably 1) amino acids; (iii) the amino acid sequence as shown in SEQ ID NO: 2 with insertion of 1 to 5 (preferably 1 to 3, more preferably 1 to 2, still more preferably 1) amino acids; (iv) the amino acid sequence as shown in SEQ ID NO: 2 with substitution of 1 to 5 (preferably 1 to 3, more preferably 1 to 2, still more preferably 1) amino acids with other amino acids; and (v) an amino acid sequence which is a combination of the above sequences (i) to (iv) may be enumerated. (Mark et al. (1984) Proc. Natl. Acad. Sci. USA 81: 5662-6; Zoller and Smith (1982) Nucleic Acids Res. 10: 6487-500; Wang et al. (1984) Science 224: 1431-3; Dalbadie-McFarland et al. (1982) Proc. Natl. Acad. Sci. USA 79: 6409-13).

Herein, the substitution of amino acid means a mutation in which one or more amino acid residues in a sequence are substituted with different kind of amino acid residues. When an amino acid sequence of the present invention is altered by such substitution, it is preferable to perform conservative substitution if it is necessary to retain the function of the protein. Conservative substitution denotes changing a sequence so as to encode an amino acid having a similar property as that of the amino acid before substitution. Amino acids can be classified by their properties into, for example, nonpolar amino acids (Ala, Ile, Leu, Met, Phe, Pro, Trp, Val), uncharged amino acids (Asn, Cys, Gln, Gly, Ser, Thr, Tyr), acidic amino acids (Asp, Glu), basic amino acids (Arg, His, Lys), neutral amino acids (Ala, Asn, Cys, Gln, Gly, Ile, Leu, Met, Phe, Pro, Ser, Thr, Trp, Tyr, Val), aliphatic amino acids (Ala, Gly), branched amino acids (Ile, Leu, Val), hydroxyamino acids (Ser, Thr), amide-type amino acids (Gln, Asn), sulfur-containing amino acids (Cys, Met), aromatic amino acids (His, Phe, Trp, Tyr), heterocyclic amino acids (His, Trp), imino acids (Pro, 4Hyp), and the like.

Therefore, it is preferable to substitute among non-polar amino acids or among uncharged amino acids. Among all, substitutions among Ala, Val, Leu, and Ile, between Ser and Thr, between Asp and Glu, between Asn and Gln, between Lys and Arg, and between Phe and Tyr are preferable for retaining the property of a protein. There is no particular limitation to the number and site of amino acids to be mutated.

Amino acid residues constituting the protein of the present invention may be naturally occurring residues or modified residues. Examples of the modification of amino acid residues include acylation, acetylation, amidation, arginylation, GPI anchor formation, cross-linking, γ-carboxylation, cyclization, formation of a covalent cross-linkage, glycosylation, oxidation, covalent binding of a lipid or fat derivative, formation of a disulfide bond, selenoylation, demethylation, decomposition of a protein, covalent binding of a nucleotide or a nucleotide derivative, hydroxylation, formation of pyroglutamate, covalent binding of flavin, prenylation, covalent binding of a heme moiety, covalent binding of phosphatidylinositol, formylation, myristoylation, methylation, ubiquitination, iodination, racemization, ADP-ribosylation, sulfation, phosphorylation, etc. Furthermore, the protein of the present invention includes a fusion protein in which a peptide sequence is added to the protein of the present invention. As the peptide sequence to be added to the protein of the present invention, a sequence facilitating the discrimination of the protein, such as influenza agglutinin (HA), glutathione S transferase (GST), substance P, poly histidine tag (6 x His, 10 x His, etc.), protein C fragment, maltose binding protein (MBP), immunoglobulin constant region fragment, α-tubulin fragment, β-galactosidase, B-tag, c-myc fragment, E-tag (an epitope on a monoclonal phage), FLAG (Hopp et al. (1988) Bio/Technol. 6: 1204-10), lck tag, p18 HIV fragment, HSV-tag (human herpes simplex virus glycoprotein), SV40T antigen fragment, T7-tag (T7 gene 10 protein), VSV-GP fragment (Vesicular stomatitis virus glycoprotein), etc.; a sequence giving stability when the protein is produced by recombinant techniques; and the like can be selected.

### <Tannase Gene>

Generally, tannase gene means a polynucleotide comprising a nucleotide sequence encoding tannase. The tannase gene of the present invention (hereinafter, sometimes referred to as the "polynucleotide of the present invention") encompasses polynucleotides having a nucleotide sequence identical or substantially identical to the nucleotide sequence as shown in SEQ ID NO: 1. As polynucleotides having a nucleotide sequence substantially identical to the nucleotide sequence as shown in SEQ ID NO: 1, any polynucleotide having a nucleotide sequence encoding the above-described protein of the present invention may be given without particular limitation. For example, in addition to polynucleotides encoding the amino acid sequence as shown in SEQ ID NO: 2, polynucleotides encoding a mutated protein that consists of the amino acid sequence as shown in SEQ ID NO: 2 with deletion, insertion, substitution or addition of one or several amino acids and has a tannase activity may be enumerated.

The term "polynucleotide" as used herein refers to a polymer composed of a plurality of bases or base pairs such as deoxyribonucleic acid (DNA) or ribonucleic acid (RNA), and includes DNA, cDNA, genomic DNA, chemically synthesized DNA, and RNA. The term "polynucleotide" also includes polynucleotides containing, if necessary, bases other than naturally occurring bases, such as 4-acetylcytidine, 5-(carboxyhydoxymethyl)uridine, 2'-O-methylcytidine, 5-carboxymethylaminomethyl-2-thiouridine, 5-carboxymethylaminomethyluridine, dihydrouridine, 2'-O-methylpseudouridine, β-D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimehtylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, β-D-mannosylqueuosine, 5-methoxycarbonylmethyl-2-thiouridine, 5-methoxycarbonylmethyluridine, 5-methoxyuridine, 2-methylthio-N-6-isopentenyladenosine, N-((9-β-D-ribofuranosyl-2-methylthiopurin-6-yl)carbamoyl)threonine, N-((9-β-D-ribofuranosylpurin-6-yl)-N-methylcarbamoyl)-threonine, uridine-5-oxyacetic acid-methyl ester, uridine-5-oxyacetic acid, wybutoxosine, pseudouridine, queuosine, 2-thiocytidine, 5-methyl-2-thiouridine, 2-thiouridine, 4-thiouridine, 5-methyluridine, N-((9-β-D-ribofuranosylpurin-6-yl)carbamoyl)threonine, 2'-O-methyl-5-methyluridine, 2'-O-methyluridine, wybutosine, 3-(3-amino-3-carboxypropyl)uridine, and the like.

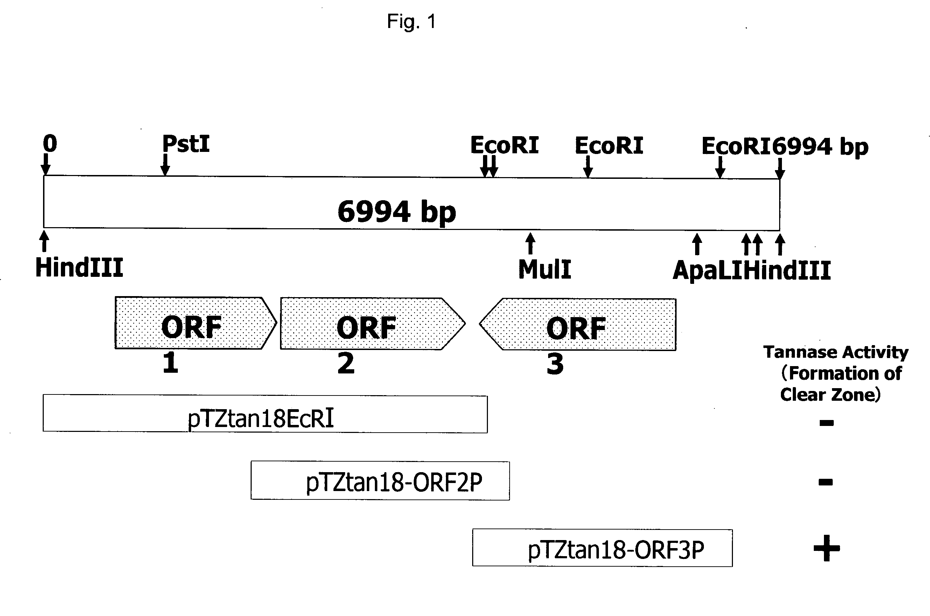

The tannase gene of the present invention corresponds to the ORF3 region shown in Fig. 1 (SEQ ID NO: 1), and comprises a nucleotide sequence encoding the amino acid sequence as shown in SEQ ID NO: 2. Such a nucleotide sequence encoding the amino acid sequence includes, in addition to the nucleotide sequence as shown in SEQ ID NO: 1, a nucleotide sequence different therefrom due to the degeneracy of genetic codes. In addition to the nucleotide sequence as shown in SEQ ID NO: 1, a polynucleotide encoding coding regions alone may also be used. When the polynucleotide of the present invention is used for expressing a protein by genetic engineering techniques, a nucleotide sequence with high expression efficiency can be selected and designed by taking into consideration the codon usage frequency in a host cell to be used (Grantham et al. (1981) Nucleic Acids Res. 9: 43-74).

Furthermore, the tannase gene of the present invention encompasses a polynucleotide that hybridizes to the nucleotide sequence as shown in SEQ ID NO: 1 or a sequence complementary thereto under stringent conditions. As such a polynucleotide, isoforms, alternative isoforms and allelic variants are conceived, and they are included in the tannase gene of the present invention. Such a tannase gene can be obtained by a known hybridization method such as colony hybridization, plaque hybridization, or Southern blotting, using the polynucleotide consisting of the nucleotide sequence as shown in SEQ ID NO: 1 or a fragment thereof as a probe. For these methods, "Molecular Cloning, A Laboratory Manual 2nd ed." (Cold Spring Harbor Press (1989)) can be referred to.

More specifically, the tannase gene of the present invention may be obtained by the method as described below. First, a tannase-producing bacterium with a tannase gene is cultured in an appropriate medium. As the medium, a medium for anaerobic culture, such as Brain Heart Infusion medium (GHI: Difco), may be used. Culture conditions may be selected appropriately depending on the required amount of DNA. For example, the bacterium may be cultured overnight at 37°C under shaking. Alternatively, the above culture may be performed using a tannase-producing bacterium isolated from colonies surrounded by a clear zone on tannic acid-treated agar medium.

For DNA extraction, cells are harvested by centrifuging the resultant culture broth, and the cell pellet is lysed so that DNA is eluted out of the cells. For this lysis treatment, a known method may be used. For example, the cell pellet is suspended in a cell resuspension solution [50 mM Tris (pH 7.5), 10 mM EDTA 2Na, 100 µg/mL RNase A]. Then, achromopeptidase-containing cell resuspension solution is added thereto to treat the suspension at 50°C for 30 minutes. Thereafter, sodium lauryl sulfate (SDS) is added thereto to thereby lyse cells. Then, phenol-chloroform solution [equilibrated phenol 50 mL, chloroform 48 mL, isoamyl alcohol 2 mL, 0.1 M Tris-HCl (pH 8.0) 10 mL] is added and mixed well. After centrifugation under appropriate conditions, the aqueous layer (the supernatant) was collected. Ethanol is added to the supernatant, which is further centrifuged so that chromosomal DNA can be collected as precipitate. The thus obtained DNA may be integrated into an appropriate vector.

Stringent conditions in the hybridization condition of the present invention are, for example, "2 x SSC, 0.1% SDS, 50°C", "2 x SSC, 0.1% SDS, 42°C", or "1 x SSC, 0.1% SDS, 37°C". More stringent conditions are, for example, "2 x SSC, 0.1% SDS, 65°C", "0.5 x SSC, 0.1% SDS, 42°C", "0.2 x SSC, 0.1% SDS, 65°C", and the like. More specifically, as a method using Rapid-hyb buffer (Amersham Life Science), the following method can also be conceived. That is, prehybridization is performed at 68°C for 30 minutes or more. Thereafter, a probe is added and kept at 68°C for one hour or more to form a hybrid. Then, the hybrid is washed three times at room temperature for 20 minutes in 2 x SSC, 0.1% SDS, washed three times at 37°C for 20 minutes in 1 x SSC, 0.1% SDS, and finally washed twice at 50°C for 20 minutes in 1 x SSC, 0.1% SDS. Alternatively, the following may also be performed. For example, prehybridization is performed at 55°C for 30 minutes or more in Expresshyb Hybridization Solution (CLONTECH). A labeling probe is added. The mixture is incubated at 37°C to 55°C for one hour or more. Then, the hybrid is washed three times at room temperature for 20 minutes in 2 x SSD, 0.1% SDS, and washed once at 37°C for 20 minutes in 1 x SSC, 0.1% SDS. Herein, for example, by increasing the temperature in prehybridization, hybridization, and the second and subsequent washing, more stringent conditions can be obtained. For example, the temperature of prehybridization and hybridization can be set at 60°C, and can be set at 68°C as more stringent conditions. Those skilled in the art could appropriately set various conditions for obtaining a polynucleotide encoding the tannase gene of the present invention, such as probe concentration, probe length and reaction time, in addition to the conditions such as salt concentration and buffer temperature.

With respect to the detailed procedures of hybridization methods, "Molecular Cloning, A Laboratory Manual 2nd ed." (Cold Spring Harbor Press (1989); particularly, Section 9.47-9.58), "Current Protocols in Molecular Biology" (John Wiley & Sons (1987-1997); particularly, Section 6.3-6.4), "DNA Cloning 1: Core Techniques, A Practical Approach 2nd ed." (Oxford University (1995); particularly, Section 2.10 regarding the conditions), etc. can be referred to. Examples of hybridizable polynucleotides include a polynucleotide comprising a nucleotide sequence having at least 50% or more, preferably 70%, more preferably 80%, and still more preferably 90% (e.g., 95% or more, or 99%) identity with the nucleotide sequence as shown in SEQ ID. NO: 1 or a nucleotide sequence complementary thereto. Such identity can be determined with BLAST algorithm (Altschul (1990) Proc. Natl. Acad. Sci. USA 87: 2264-8; Karlin and Altschul (1993) Proc. Natl. Acad. Sci. USA 90: 5873-7). As programs based on this algorithm, BLASTX and the like have been developed as programs for determining identity in amino acid sequences; and BLASTN and the like have been developed as programs for determining identify in nucleotide sequences (Altschul et al. (1990) J. Mol. Biol. 215: 403-10). These programs can be used for the sequence of the present invention. For specific analysis methods, see, for example, http://www.ncbi.nlm.nih.gov.

Genes that have a structure and a function similar to those of tannase gene (such as isoforms, alternative isoforms and allelic variants of tannase gene included in the tannase gene of the present invention) may be obtained by designing primers based on the nucleotide sequence as shown in SEQ ID NO: 1 and using a gene amplification technique (PCR) (Current Protocols in Molecular Biology, John Wiley & Sons (1987) Section 6.1-6.4). In this case, any part of the nucleotide sequence as shown in SEQ ID NO: 1 may be used as primers. For example, Tan-F-F6: 5'- cgtggtgccctattacaagt -3' (SEQ ID NO: 14) may be used as a upstream primer and Tan-F-R7: 5'- tgagccttcagtatattgacca -3' (SEQ ID NO: 15) may be used as a downstream primer. Then, PCR is performed using *S*. *lugdunensis* genomic DNA, the above primers and DNA polymerase. The resultant PCR product may be purified by known methods, such as a method using ethidium bromide, a method using SYBR GreenI (Molecular Probes), a method using agarose gel such as GENECLEAN (Funakoshi) or QIAGEN (QIAGEN), a method using DEAE-cellulose filter, the freeze and squeeze technique, a method using a dialysis tube, etc. When an agarose gel is used, the PCR product is electrophoresed on the agarose gel and then resultant DNA fragments are cut out from the gel and purified.

The polynucleotide of the present invention encompasses polynucleotides encoding the amino acid sequence as shown in SEQ ID NO: 2 with deletion, insertion, substitution or addition of one or more amino acids; and sequences complementary to the sequences of such polynucleotides. These polynucleotides of the present invention may be prepared according to site-directed mutagenesis methods or the like described, for example, in "Molecular Cloning, A Laboratory Manual 2nd ed." (Cold Spring Harbor Press (1989)); "Current Protocols in Molecular Biology" (John Wiley & Sons (1987-1997); especially Section 8.1-8.5); Hashimoto-Goto et al. (1995) Gene 152: 271-5; Kunkel (1985) Proc. Natl. Acad. Sci. USA 82: 488-92; Kramer and Fritz (1987) Method. Enzymol. 154: 350-67; and Kunkel (1988) Method. Enzymol. 85: 2763-6.

Introduction of mutations into polynucleotides may be performed according to known techniques, such as Kunkel's method or gapped duplex method, using a mutation introduction kit utilizing site-directed mutagenesis, e.g., QuikChange^{™} Site-Directed Mutagenesis Kit (Stratagene), GeneTailor^{™} Site-Directed Mutagenesis System (Invitrogen), TaKaRa Site-Directed Mutagenesis System (Mutan-K, Mutan-Super Express Km, etc.: Takara Bio), or the like.

The nucleotide sequence of the polynucleotide of the present invention may be confirmed by sequencing according to a conventional method such as the dideoxynucleotide chain termination method (Sanger et al. (1977) Proc. Natl. Acad. Sci. USA 74: 5463). Alternatively, it is also possible to analyze the sequence of the polynucleotide with an appropriate DNA sequencer.

### <Preparation of Tannase Protein Expression Vector and Transformation with the Vector>

In order to amplify the region of the thus sequenced nucleotide sequence encoding the tannase protein, two primers having different restriction enzyme sequences are designed; one is located 5' to the initiation codon and the other is located 3' to the termination codon. Gene amplification is performed, and the resultant amplified region is cut out with restriction enzymes and integrated into an expression vector, which is then introduced into a host (*Escherichia coli* or the like). Thus, a transformant can be obtained.

Specifically, a plasmid comprising a polypeptide encoding the protein of the present invention cloned from the PCR product is digested with restriction enzymes. If there are appropriate restriction enzyme sites in the tannase gene sequence, restriction enzymes corresponding thereto may be used. Alternatively, restriction enzymes corresponding to the restriction enzyme sequences inserted into PCR primers may be used. For example, when primers ORF3-F (BamHI) (SEQ ID NO: 10) and ORF3-R (XhoI) (SEQ ID NO: 11) are used, restriction enzymes BamHI and XhoI may be used. The DNA fragment obtained by digestion is purified by agarose gel electrophoresis. This DNA fragment is integrated into an expression vector by a known method to thereby obtain a tannase expression vector. This expression vector is introduced into a host to prepare a transformant, which is used for expression of the gene of interest. Here, the expression vector and the host are not particularly limited, as long as they can express the gene of interest. For example, a bacterium such as *Escherichia coli* or *Bacillus subtilis;* a yeast such as *Saccharomyces cerevisiae;* or a mammal cell such as COS cell or CHO cell may be used as the host. When a bacterium such as *E*. *coli* is used as the host, it is preferable that the expression vector comprises a promoter and a transcription termination sequence while it ensures autonomous replication of the gene of the present invention in the host. Examples of the expression vector include pGEX (Pharmacia). As the promoter, any promoter may be used as long as it can direct expression in the host such as *E. coli.* For example, an *E. coli-* or phage-derived promoter such as trp promoter, lac promoter, PL promoter, PR promoter or the like may be used. As a method for introducing a recombinant DNA into a bacterium, electroporation, the calcium chloride method or the like may be used. When a yeast is used as the host, an expression vector such as YEp13 or YCp50 may be used. As a promoter, gall promoter or gal10 promoter may be used. As a method for introducing a recombinant vector into yeast, electroporation, the spheroplast method, the lithium acetate method or the like may be used. When a mammal cell is used as the host, an expression vector such as pcDNA3 (INVITROGEN CORPORATION) may be used. TIGAI expression vector plasmid is introduced into a cell strain, for example, by the liposome method using Lipofectamine (GIBCO BRL).

The host cell into which the tannase gene has been introduced as described above is cultured in a tannic acid-treated agar medium. When a clear zone is observed around the resultant colony, it can be judged that the colony has the tannase gene cloned therein. For example, transformed *E*. *coli* cells are cultured in a tannic acid-treated agar medium, and colonies surrounded by a clear zone are selected. It is possible to determine the nucleotide sequence of the tannase gene from the vector carried by the selected clone. Briefly, after cloning of the purified DNA fragment as described above, whether the cloned PCR product is the open reading frame (ORF) of interest or not is decided by determining the nucleotide sequence with a DNA sequencer.

### <Purification of Tannase>

Tannase may be purified from a supernatant obtained by culturing in a large scale a transformant prepared by integrating a tannase protein expression vector into a host (E. *coli,* etc.), disrupting the resultant culture with surfactants and ultrasounds and then centrifuging the thus treated culture. The purification may be performed by a combination of various chromatographies (e.g., affinity chromatography, ion exchange chromatography and gel filtration chromatography) with tannase activity as an indicator.

Specifically, the above-described colony in which the tannase gene has been cloned is cultured, for example, in ampicillin (Amp)-containing Luria medium (LB) (LB-Amp medium) and harvested. The cells are suspended in a buffer such as PBS(-) that is prepared to have an appropriate pH for retaining the enzyme stable. Into the buffer, a protease inhibitor such as PMSF (phenylmethanesulfonyl fluoride) and lysozyme with a lytic effect are added and incubated for lysis. The resultant supernatant is used as a crude enzyme solution. The protease inhibitor is used for preventing the degradation of the enzyme of the present invention by the action of the co-existing protease during the process of extraction of the enzyme of the present invention. The crude enzyme solution is thermally treated and centrifuged, followed by collection of the supernatant. Thus, *E. coli*-derived proteins are removed. After dialysis, this supernatant is applied to a cation or anion exchange column to allow elution of the protein of interest with linear concentration gradient using sodium chloride, etc. Out of the eluted fractions, the fraction with the highest specific activity is concentrated by ultrafiltration and purified by high performance liquid chromatography, to thereby obtain the tannase of the present invention.

Alternatively, in the present invention, it is also possible to obtain tannase from the tannase gene or the above-described vector. Briefly, it is possible in the present invention to produce tannase using a cell-free protein synthesis system without any use of living cells.

The term "cell-free protein synthesis system" refers to a system in which a protein is synthesized in an artificial vessel such as a test tube using a cell extract. For example, mRNA information is read, and a protein is synthesized on ribosomes. It should be noted that the cell-free protein synthesis system used in the present invention also encompasses a cell-free transcription system which synthesizes RNA using DNA as a template.

As the above cell extract, a eukaryote- or prokaryote-derived extract may be used. For example, an extract from wheat germs, rabbit reticulocytes, mouse L-cells, HeLa cells, CHO cells, budding yeast, E. *coli* or the like may be used. These cell extracts may be either concentrated extracts or non-concentrated extracts.

Here, the genetic information encoded on DNA is converted to mRNA through transcription and further converted to a protein through translation. In order to reproduce this translation process in an artificial vessel to synthesize a protein, it is necessary to stabilize a group of translation factors (such as ribosome, tRNA, and various protein synthesis factors) and to construct a system that produces mRNA depending on the purpose. For the construction of such a system, wheat germs may be selected in the present invention. Wheat germs are preferable because a group of translation factors necessary for seeds to bud and perform protein synthesis are preserved therein.

The cell extract may be obtained by methods such as ultrafiltration, dialysis, polyethylene glycol (PEG) precipitation, etc. In the method by PEG precipitation, a cell extract and PEG solution are mixed to precipitate nucleic acid. The nucleic acid is collected and dissolved in a small amount of buffer to thereby obtain a concentrated cell extract. Concentration by dialysis may be performed, for example, in a closed system capable of shaking or agitation and by placing the cell extract against a dialysis external liquid through a dialysis membrane (e.g., cut off molecular weight: 1000-10000). Here, the dialysis external liquid may comprise a buffer (containing potassium acetate, magnesium acetate, dithiothreitol, or the like) and a polymer absorber (e.g., PEG, Ficoll, or the like).

In the present invention, it is also possible to perform cell-free protein synthesis using a commercial kit. Examples of such kits include a reagent kit PROTEIOS^{™} (Toyobo) and a synthesis device PG-Mate^{™} (Toyobo).

Tannase obtained by cell-free protein synthesis may be purified by selecting appropriate chromatographies as described above.

The isolation and purification of tannase may be confirmed by SDS-PAGE (Laemmli, U. K., Nature, 227, 680-685, 1970), and the activity of the purified tannase may be measured by HPLC. Specifically, tannase activity may be measured, for example, according to the method disclosed in WO 02/34938 by reacting a tannase sample with methyl gallate (substrate) and quantitatively determining the amount of gallic acid (degradation product) by HPLC. Alternatively, tannase activity may be measured by mixing methyl gallate with a tannase sample and measuring absorbance at 440 nm. Alternatively, tannase activity may be measured by mixing methyl gallate with a tannase sample and observing if the color of the mixed solution turns green and then brown. Preferably, the activity is quantitatively determined by measuring absorbance.

### <Preparation of Antibodies to Tannase>

Polyclonal antibodies to tannase may be obtained by immunizing a non-human animal (e.g., domestic rabbit) with a purified tannase. It is also possible to obtain monoclonal antibodies by immunizing an animal such as mouse with a purified tannase and fusing spleen cells from the animal with myeloma cells. Hereinbelow, preparation of such antibodies will be described in detail.

The term "antibody" used herein means the entire antibody molecule (either polyclonal or monoclonal antibody) that can bind to an antigen tannase or a partial fragment thereof. Further, the term "antibody" also means a fragment of the antibody molecule, i.e., an active fragment having antigen-antibody reaction activity (specifically, Fab, F(ab')₂, Fv, recombinant Fv, single chain Fv, or the like).

### (1) Preparation of Antigen

As an antigen for preparing the above antibody, the protein of the present invention as described above or a protein substantially identical thereto may be used. The term "protein substantially identical" used herein encompasses proteins different from the protein of the present invention in terms of presence/absence or type/degree of artificial or physiological modifications (such as sugar chains) or labels (such as fluorescence or radioactive substances). That is, depending on the host that expresses a protein, modification of the resulting protein with sugar chains may be different from that of the protein of the present invention. However, even though the resulting protein has difference in sugar chain modification, the protein is regarded as tannase or a protein substantially identical to tannase and may be used as an antigen, if the protein shows properties similar to those of tannase.

Further, it should be noted that, in the detection of antibodies, not only an antigen molecule itself (i.e., tannase or a protein substantially identical thereto) but also a complex comprising a fragment, fragment peptide or a chemically synthesized oligopeptide of such proteins together with an appropriate carrier are often used as antigen. The reason for that is because an analytical system specific to a particularly dominant epitope or a clinically significant epitope will be less affected by non-specific reactions. This means that the cloning of hybridomas and clone selection described later can be performed more efficiently and that anti-tannase monoclonal antibodies of higher antibody titers can be obtained. With respect to epitope sites, it is more preferable to allow antibodies to recognize a smaller number of specific structures of the antibody protein in the lower structure than a large number of regions in the higher structure of the antibody protein.

Such an approach is also effective in obtaining monoclonal antibodies with high specificity and affinity to tannase. Specifically, domains capable of functioning as epitopes may be determined by the method of obtaining immunologically active domain peptides described later.

It is known that an epitope may be composed of at least 3 amino acid residues. It is also known that immunological discrimination from other proteins becomes possible with at least 6 amino acid residues. Therefore, an epitope consists of at least 6 consecutive amino acid residues, usually 8 amino acid residues, preferably 10 amino acid residues, still preferably 11-15 amino acid residues selected from the amino acid sequence of tannase or a protein substantially identical thereto.

Further, methods of improving the immunological reactivity of epitopes by variously modifying oligopeptides constituting the epitopes are known to those skilled in the art. For example, modifications such as addition of an inactive protein (e.g., human serum albumin) or a nonsense amino acid sequence contributes to improvement of immunological reactivity.

Fragments of tannase may be prepared by digesting with enzymes such as trypsin, chymotrypsin or pepsin, by chemically cutting with cyanogen bromide, or by a combination of these methods. In order to isolate and purify a peptide of interest from a resultant peptide mixture, known separation technologies such as chromatography, electrophoresis or the like may be used.

Alternatively, tannase fragments may be obtained by cutting a DNA encoding tannase at random, inserting the resultant fragments into phage vector and preparing phage libraries presenting domain peptides. Immunologically active domains may be determined by immuno-screening these libraries with an antibody that recognizes tannase. Alternatively, first, a peptide portion consisting of 14 or more amino acid residues which is believed to be appropriate is determined by a method of analysis searching the hydrophilic profile of tannase and other immunogenic indexes. Such peptides as candidate immunogens may be synthesized by a known peptide synthesis technique, liquid phase synthesis or solid phase synthesis. The solid synthesis method represented by the Merrifield method enables simple and prompt synthesis.

As an α-amino protective group, normally, t-butoxycarbonyl (Boc) is used. 9-Fluorenylmethoxycarbonyl (Fmoc) group developed by Sheppard et al. (Atherton, E. and Sheppard, R.C. (1985) J. Chem. Soc. Chem Comm.165) may also be used (Nobuo Izumiya et al., "Fundamentals and Experiments in Peptide Synthesis", pp. 194-233, Maruzen, 1985).

In the present invention, an automated peptide synthesizer based on the solid phase synthesis method may be used. The synthesized peptide is cut out from the resin in the presence of trifluoroacetate or the like, and then purified by reversed phase high performance liquid chromatography. It is desired that the purified peptide should have 85% or more purity.

The above-described candidate peptides are used to immunize animals. Polyclonal antibodies collected from these immunized animals are reacted with tannase and then subjected to a detection reaction using enzyme-labeled anti-IgG antibody. Those peptides which show positivity in the detection reaction may be used as the immunizing antigen for preparing the antibody of the present invention.

An immunogen prepared by attaching such a peptide to a carrier protein is specific to tannase and yields antibodies with sufficient binding affinity. For example, the above-described peptide of tannase that is a useful immunogen may be attached to keyhole limpet hemocyanin (KLH) as a carrier protein and then used for immunizing non-human animals.

Examples of other carrier substances which may be used to obtain immunogens include tuberculin purified protein derivatives, tetanus denatured toxin, cholera toxin and B subunit thereof, diphtheria toxin, ovalbumin, bovine serum albumin, soybean trypsin inhibitor, muramyl dipeptide, and Brown's lipoprotein. With respect to reactions, reagents, etc. for attaching peptides to carrier proteins, those disclosed in literature may also be used.

### (2) Preparation of Polyclonal Antibodies to Tannase

An antigen prepared as described above is administered to a mammal. The mammal is not particularly limited. For example, mouse, guinea pig, rabbit, rat, sheep, goat, monkey, dog or the like may be used. Particularly preferable is mouse or rabbit because they are easy to handle.

The dose of antigen per animal is, for example, 1-10 mg in the case of rabbit when no adjuvant is used. When adjuvant is used, the dose is 0.01-1 mg, for example. Examples of adjuvants include Freund's complete adjuvant (FCA), Freund's incomplete adjuvant (FIA) and aluminium hydroxide adjuvant. Immunization may be performed by injecting the antigen intravenously, subcutaneously, intraperitoneally, etc. The immunization interval is not particularly limited. Immunization may be carried out at appropriate intervals of several days to several weeks. Then, antibody titers are determined by such methods as ELISA (enzyme-linked immunosorbent assay) or EIA (enzyme immunoassay), radioimmuno assay (RIA) or the like. Blood are collected on the day when highest antibody titers are shown, to thereby obtain antisera.

### (3) Preparation of Monoclonal Antibodies to Tannase

### (i) Collection of Antibody-Producing Cells

For preparation of monoclonal antibodies, an immunizing antigen (i.e., tannase or a protein immunologically equivalent thereto, or a fragment or fragment peptide thereof) is administered to a mammal at a site capable of antibody production, alone or together with a carrier and a diluent. At that time, adjuvant or the like may be added to the antigen in order to enhance its antibody-producing ability (Adv. Tubercl. Res., 1:130-148, 1956). Examples of adjuvants include Freund's complete adjuvant (FCA), Freund's incomplete adjuvant (FIA) and aluminium hydroxide adjuvant.

Monoclonal antibodies may be obtained by allowing formation of a fusion cell between an immunocompetent cell (i.e., antibody-producing cell) and a myeloma cell, cloning the fusion cell, and selecting those clones producing tannase-specific antibodies (Kohler, G & Milstein, C., Nature 256: 495-7, 1975).

For immunization, a complex comprising the above-described peptide (or tannase or a protein immunologically equivalent thereto, or a fragment or fragment peptide thereof) is used as an immunogen. For example, 20 µg-1 mg of the immunogen per administration may be dissolved in or mixed with an appropriate adjuvant and used for immunizing an animal.

As the mammal to be immunized, mouse, guinea pig, rat, monkey or the like may be enumerated. Especially preferable is mouse or rat because they are easy to handle. It is desirable that the antibody-producing cell and the myeloma cell are derived from the same species of animal. Usually, administration may be performed once in every 2 to 6 weeks, about 2 to 10 ten times over 3 to 6 months, but is not limited to this manner.

Collection of antibody-producing cells may be performed by selecting individuals showing antibody titers from the immunized animals and collecting from them spleen cells, lymph node cells or B lymphocytes after an appropriate time period from the final immunization.

### (ii) Cell Fusion

An antibody-producing cell obtained as described above and a myeloma cell with autonomous proliferation ability may be fused by known techniques. Basically, the procedures are according to the method of Kohler ("Immunological Method", Academic Press, New York, 391, 1979). Examples of myeloma cells include, but are not limited to, mouse myeloma-derived cell strains such as P3U1, NS-1, SP2/0 and mutants thereof Examples of cell fusion promoters include, but are not limited to, polyethylene glycol (PEG) and Sendai virus. Cell fusion may be performed, for example, by suspending myeloma cells in a serum-free liquid medium, adding to this suspension a liquid containing antibody-producing cells (e.g., spleen cells) prepared above, leaving the resultant mixture stationary for a while, centrifuging the mixture lightly to collect cells, adding a PEG-containing medium to the cells, and then incubating for several minutes. By these operations, cell fusion is completed.

Hybridomas which were collected by centrifuging the reactant are transferred to an appropriate selection medium (e.g., HAT medium), seeded in 96-well microplates, and cultured for a specific period of time. Culture conditions are not particularly limited. For example, hybridomas are cultured in an incubator under 5% CO₂ at 20-40°C, preferably at 34-38 °C. Usually, hybridomas grow and form colonies after 1 week from the start of culture.

### (iii) Antibody Screening and Cloning

Selection of monoclonal antibodies may be performed usually in HAT (hypoxanthine, aminopterin and thymidine)-added animal cell culture broth. The medium for selection and breeding is not particularly limited as long as hybridomas are capable of growing therein. Example of useful media include serum-free medium for hybridoma culture, fetal calf serum (FCS)-containing RPMI medium and GIT medium.

Antibody titers to the peptide in the culture supernatants of those well where proliferation was observed are determined, for example, by enzyme antibody technique. Then, hybridomas are cloned, for example, by limiting dilution analysis to thereby obtain clones of the hybridoma of the present invention.

Operations for screening for the hybridoma of the present invention formed are as described below but are not limited to these operations. Briefly, a culture supernatant of a hybridoma is added to a solid phase (e.g., microplate) onto which the peptide used as an immunogen has been adsorbed directly or together with a carrier. Then, anti-immunoglobulin antibody labeled with a radioactive substance, enzyme, etc. or protein A is added thereto to detect the monoclonal antibody bound to the solid phase. This method is convenient. Alternatively, a culture supernatant of a hybridoma may be added to a solid phase onto which anti-immunoglobulin antibody or protein A has been adsorbed, and then a labeled antigen may be added to detect the monoclonal antibody bound to the solid phase.

Positive wells are selected by the above-described operations. Several days thereafter, each clone is seeded in one 96-well plate to give a density of, for example, 100 cells/plate. After several days culture, colonies are judged. The culture supernatant is applied to the above-described antigen-immobilized plate for screening, to thereby perform an assay in the same manner. The selected colonies are cultured and re-cloned. After several days culture, judgment of colonies and assay of culture supernatant can be performed in the same manner as described above. Wells are selected by parent clones and cultured; the supernatants are collected for checking clones; thus, antibody sub-classes and antibody production can be assayed.

### (iv) Production and Isolation of Monoclonal Antibodies

When the thus selected clone is cultured *in vitro* or *in vivo,* the monoclonal antibody of the present invention is produced. For this culture, conventional methods in the relevant technical field may be used.

When a hybridoma is cultured *in vitro* in a culture medium, a monoclonal antibody may be isolated from the resultant culture. In order to improve proliferation rate and antibody production efficiency, selection and maintenance of an appropriate medium and administration of culture conditions (e.g., oxygen concentration in the medium, agitation speed, etc.) are required.

When a clone is cultured in a warm-blooded animal other than human, a monoclonal antibody may be collected from the abdominal dropsy and/or blood of the animal. For example, the clone is inoculated into fetal calf serum-supplemented RPMI1640 medium to give a specific cell density and cultured in an incubator in the presence of 5% CO₂. Subsequently, the resultant culture is inoculated into the peritoneal cavity of pristane-treated mice, which are then bred by a conventional method for a specific period of time. After several weeks, the abdominal dropsy is collected. Ammonium sulfate is added thereto for salting out to thereby obtain a precipitate. The monoclonal antibody of interest may be isolated and purified from this fraction by means of chromatography, etc.

The method of isolating monoclonal antibodies from abdominal dropsy and/or blood is the same as the known method of purifying polyclonal antibodies, and the isolation/purification method for immunoglobulin may be used. That is, salting out, dialysis, filtration, concentration, alcohol precipitation, isoelectric precipitation, various electrophoresis methods, adsorption/desorption with ion exchangers (e.g., DEAE resin), ultracentrifugation, gel filtration, specific affinity purification, and so on may be used in an appropriate combination. The monoclonal antibody produced is then appropriately concentrated, and may be used in a liquid form or a solid form via freeze-drying, depending on the use.

### <Application of Tannase Detection Method to Diagnosis of Colon Cancer>

Tannase-producing bacteria are found in feces of a large number of colon cancer patients. Since isolation ratios of tannase-producing bacteria from feces are high in colon cancer patients among those patients who underwent endoscopy of the lower gastrointestinal tract, it is reported that tannase-producing bacteria are involved in human colon cancer. Therefore, by subjecting tannase genes isolated from tannase-producing bacteria in human feces to genetic engineering techniques or by using immunological properties of anti-tannase antibodies, there are provided a method of detecting colon cancer and a method of diagnosing colon cancer.

### (1) Immunological Method of Detecting Colon Cancer

### (1-1) Method of Detecting Colon Cancer with Antibody

The method of detecting colon cancer according to the present invention comprises: a step of reacting a biological sample from a subject with the antibody of the present invention or a fragment thereof to thereby detect a disease marker in the sample; and a step of relating the detection results from the above step to the disease. Further, the present invention also encompasses a method of detecting tannase-producing *Staphylococcus lugdunensis* comprising reacting a test sample with the antibody of the present invention or a fragment thereof

As the above-mentioned disease marker, any of the following markers may be used. Methods of measuring these markers will be described later.
(a) tannase or a protein substantially identical thereto, or
(b) tannase or a peptide substantially identical thereto

It is possible to measure the tannase produced by tannase-producing bacteria (in particular *Staphylococcus lugdunensis)* in a test sample (e.g., human feces) by immunochemical techniques using the antibody to tannase (anti-tannase antibody) prepared by the present invention. For example, when tannase or a protein substantially identical thereto has been detected in a fecal sample taken from a subject, it is beliebed that tannase-producing bacteria (in particular *Staphylococcus lugdunensis)* are present in the enteric bacteria of the subject and that the subject is affected by colon cancer at a high possibility.

In other words, the expression of tannase or a protein substantially identical thereto in feces collected from a subject indicates that the subject is affected by colon cancer. Generally, it is possible to detect the expression of a protein using the presence of the protein itself or mRNA carrying the information thereof as an indicator. For detection of tannase, the anti-tannase antibody of the present invention is preferably used. When tannase is detected in feces from a subject, it is highly possible that the subject is affected by colon cancer.

The antibody of the present invention may be used for the preparation of antibody columns for tannase purification, or for the detection of the protein in individual fractions during purification. However, the application of the specific anti-tannase antibody of the present invention is not limited to these uses.

### (1-2) Application as Diagnostic Reagent of Colon Cancer Comprising the Antibody

Subsequently, in what embodiment an immunological diagnostic reagent for colon cancer comprising the anti-tannase antibody is used for the above-described use will be described specifically below.

With respect to immunological techniques to determine the antigen or antibody in test samples, known methods may be applicable. A measurement method may also be used in which the amount of antibody or antigen-antibody complex corresponding to the amount of antigen (i.e., tannase protein) in a sample is detected by chemical or physical means, and then the amount of antigen is calculated from a calibration curve prepared from standard solutions containing known amounts of the antigen. Examples of method which may be used include enzyme immunoassay, radio-immunoassay, chemiluminescence immunoassay, electro chemiluminescence immunoassay, immunochromatography, Western blotting, passive particle agglutination, passive hemoagglutination, and latex chemiluminescence immunoassay. From the viewpoints of sensitivity and specificity, the sandwich method (a type of enzyme immunoassay) described below is especially preferable. In the sandwich method (e.g., ELISA), a microtiter plate on which the anti-tannase antibody of the present invention is immobilized is reacted with a test sample (primary reaction) and, after washing, enzyme-labeled anti-tannase antibody is reacted as the second antibody (secondary reaction). After washing, a substrate of the enzyme is added. The amount of color development from the substrate is measured with a spectrophotometer to thereby quantitatively determine the amount of tannase or the like in the sample. In this method, the primary reaction and the secondary reaction may be performed in a reversed order, or at the same time, or at different times. The antibody used in the secondary reaction is preferably an antibody in which the tannase binding site is different from that site in the antibody of the present invention used in the primary reaction.

As the carrier for immobilizing anti-tannase antibodies on a solid phase, besides microtiter plates, any of the carriers such as magneto-sensitive beads, plastic beads, nitrocellulose membrane, nylon membrane, erythrocytes, gelatin particles, polyamino acid particles and latex particles may be used.

In the immunochromatography method using the monoclonal antibody of the present invention, the antigen in a sample and an immobilized antigen on a solid phase are allowed to compete against a specific amount of a labeled monoclonal antibody, followed by separation of the solid phase and the liquid phase. Alternatively, the antigen in a sample is reacted with an excessive amount of a labeled monoclonal antibody; then, an immobilized antigen is added to bind the unreacted labeled antibody to the solid phase; finally, the solid phase and the liquid phase are separated. Subsequently, the amount of antigen in the sample is quantitatively determined by measuring the amount of label in either one of the phase.

In the competition method, the antigen in a sample and a labeled antigen are allowed to compete against an antibody. Then, unreacted labeled antigen (F) and antibody-bound labeled antigen (B) are separated (B/F separation), and the amount of the label in either B or F is measured to thereby quantitatively determine the antigen in the sample. Alternatively, a liquid phase method may be used in which a soluble antibody is used as the antibody; polyethylene glycol is used in B/F separation; and second antibody to the above antibody is used. Alternatively, in the above method, an immobilized antibody is used as first antibody; or a soluble antibody is used as first antibody and an immobilized antibody is used as second antibody. For example, it is also possible to allow the anti-tannase antibody of the present invention insolubilized on a carrier and a labeled antibody to competitively react with a sample simultaneously or consecutively, and then measure the activity of the labeling reagent on the insolubilizing carrier.

When the anti-tannase antibody of the present invention is used as a reagent for separating or detecting cells, the anti-tannase antibody may be made into a composition containing other solvents or solutes. For example, distilled water, pH buffer reagents, salts, proteins, surfactants, or the like may be combined.

Reaction reagents have a label which is detectable by appropriate chemical or physical detection means. As a labeling reagent to be used in measuring methods using such labeling substances, a fluorescent substance, an enzyme, a radioisotope, a luminescent substance or the like may be used. When an enzyme is used for the labeling, the method is called ELISA and used widely. In this case, as the labeling reagent for second antibody, any quantifiable substances such as fluorescent substances, radioactive substances, bioluminescent or chemiluminescent substances, electrochemiluminescent substances, dyes and metals can be used, besides enzymes such as peroxidase, alkaline phosphatase and β-galactosidase.

Examples of fluorescent substances include fluorescamine and fluoresceine isothiocyanate; examples of enzymes include peroxidase, alkaline phosphatase, malate dehydrogenase, α-glucosidase and α-galactosidase; examples of radioisotopes include ¹²⁵I, ¹³¹I, ³H and ¹⁴C; examples of luminescent substances include luciferin, lucigenin, luminol and luminol derivatives. Labeling substances may be bound to antibodies by conventional operations under conventional conditions.

Further, it is also possible to use a biotin-avidin system for the binding between the anti-tannase monoclonal antibody or antigen of the present invention and a labeling reagent. For insolubilization of the antigen or monoclonal antibody, physical absorption may be used. Alternatively, chemical bonds which are usually used for insolubilization and immobilization of proteins or enzymes may be used. As a carrier, a synthetic resin such as polystyrene, polyacrylamide or silicone; an insoluble polysaccharide such as agarose, dextran or cellulose; glass or the like may be used.

As reaction media, buffers that are useful in giving optimal conditions for reaction or stabilizing reaction products; stabilizers for reactants; and the like may be enumerated.

As detection means, spectrophotometers, radiation detectors, light scattering detectors and the like which are capable of detecting the above-described label may be enumerated.

### (2) Method of Detecting Tannase Genes

The present invention also provides a polynucleotide which hybridizes to the polynucleotide of the tannase gene of the present invention under stringent conditions and has a strand length of at least 15 nucleotides. This polynucleotide may be used for detecting *Staphylococcus lugdunensis.* Therefore, the present invention provides a method of detecting *Staphylococcus lugdunensis* by contacting a test sample with a polynucleotide of the tannase gene.

The length of the polynucleotide which hybridizes under stringent conditions is at least 15 nucleotides. When used as a primer, the length of the polynucleotide is preferably 15-40 nucleotides, more preferably 20-35 nucleotides, and the hybridization conditions are usual PCR conditions. When used as a probe, the length of the polynucleotide is not particularly limited. Even a probe of several kb is allowable. The hybridization conditions are the same as described above.

Tannase genes may be detected by appropriate gene amplification operations using as a template a DNA directly obtained from human feces or obtained from bacteria in human feces cultured preferably anaerobically. For example, PCR may be performed for this purpose using primers of 15-40 nucleotides designed based on the nucleotide sequence information as shown in SEQ ID NO: 1. By such operations, tannase-producing bacteria, in particular *Staphylococcus lugdunensis* can be detected. In addition to the above PCR method, other detection techniques such as Southern blotting and Northern blotting may be used. The above-described polynucleotide is used as a primer in PCR and used as a probe in Southern blotting and Northern blotting.

Hereinbelow, the detection method will be described.

### (2-1) Method of Preparation ofPCR Samples from Human Feces

Any method may be used for collecting PCR samples from human feces. For example, samples may be collected by inserting a cotton swab into the anus of subjects. The cotton swab to which feces are attached is placed in an appropriate liquid medium and cultured. With respect to the medium and culture conditions, an anaerobic medium may be used as described above under appropriate conditions. For example, the cotton swab may be placed in *Streptococcus* selection supplement-containing Brain Heart Infusion (BHI) liquid medium and cultured overnight anaerobically at 37°C. In order to obtain a DNA sample from the resultant culture broth, known methods may be used. For example, the resultant culture broth may be mixed with sterilized water and centrifuged under appropriate conditions to precipitate the cultured cells. After removal of the supernatant, sterilized water is added to prepare a cell suspension, which is used as a DNA sample for PCR

### (2-2) Method of Amplifying Tannase Genes by PCR

The DNA sample for PCR obtained by the above-described operations, each primer and PCR Master Mix (Promega) are mixed, and sterilized purified water is added thereto to adjust the volume. As primers, polynucleotides having a strand length of at least 15 nucleotides, preferably 20 nucleotides or more are used. Specific examples of such primers include the following.

Tan-F-F6 (SEQ ID NO:14): cgtggtgccctattacaagt

Tan-F-R7 (SEQ ID NO: 15): tgagccttcagtatattgacca

Amplification of tannase genes may be performed by known methods. For example, the amplification may be performed in a thermal cycler under the following conditions. Briefly, after disruption of cells by heating at 95° for 3 minutes; 5 cycles of DNA denaturation at 95°C for 30 seconds, annealing at 50°C for 30 seconds and extension at 72°C for 30 seconds; then 25 cycles of DNA denaturation at 94°C for 20 seconds, annealing at 50°C for 20 seconds and extension at 72°C for 30 seconds; and final extension at 72°C for 5 minites are performed. The amplified product is electrophoresed in agarose X gel (Nippon Gene) of an appropriate concentration and subjected to detection of tannase genes. When the above-described primers are used, the gene is detected as a 223 bp band.

Simultaneously with the detection of tannase genes by PCR, bacteria obtained from human feces as described above may be cultured in tannic acid-over layered Brain Heart Infusion agar medium (Oxoid). Then, lysis zones showing tannase production may be confirmed, and tannase-producing bacteria may be isolated and identified.

Detection may be confirmed with one of the following results alone or a combination thereof: observation of the presence or absence of tannase genes by PCR; confirmation of lysis zones showing tannase production by a culture method; and results of whether or not *Staphylococcus lugdunensis* was isolated and identified.

### (2-3) Detection by Southern Blotting or Northern Blotting

When Southern blotting is performed, genes digested with specific restriction enzymes are electrophoresed and blotted (transferred) onto a membrane (e.g., nitrocellulose). After immobilizing the transferred nucleic acid on the membrane, the nucleic acid is hybridized to a labeled probe to detect the presence of tannase genes. For the labeling of the probe, radioisotopes of phosphorus, digoxigenin (DIG), etc. may be used.

When Northern blotting is performed, RNA extracted from a sample is developed by electrophoresis and transferred onto a membrane, followed by detection with a labeled probe.

Briefly, after nucleotides are extracted from cells with a reagent such as phenol reagent, DNA alone is digested with RNase-free DNase. The resultant nucleotides are developed on a gel by agarose gel electrophoresis or the like. This gel is transferred (blotted) onto a membrane and hybridized to a labeled nucleotide probe complementary to the RNA sequence to be detected. Thus, the amount and size of the target RNA are detected. For the labeling of the probe, digoxigenin (DIG) may be used, for example.

### (3) Diagnostic Reagent for Colon Cancer

When the method of detecting a tannase protein or the method of detecting a tannase gene according to the present invention is applied to diagnosis of colon cancer, no particular conditions or operations are required. The diagnosis is carried out under usual conditions and with usual operations in each of the above methods. If necessary, some modifications may be made to thereby construct an appropriate measuring system.

In order to enable most simple and efficient measurement, the above diagnostic reagent is made into a kit. With such a kit, it becomes possible to perform quantitative determination efficiently in an ordinary laboratory, without requiring special analysis instruments, skilled operations or high knowledge. Therefore, the present invention provides a kit for detecting *Staphylococcus lugdunensis* or a kit for detecting colon cancer, each kit comprising at least one selected from the group consisting of the antibody of the present invention or a fragment thereof, and a polynucleotide which hybridizes to the polynucleotide of the present invention under stringent conditions and has a strand length of at least 15 nucleotides. The constitution and form of the assay kit for practicing the above-described diagnosis method or method of judgment of treatment are not particularly limited. The contents of the kit are not limited as long as the kit can achieve the predetermined object. Generally, the kit is composed of a user's manual for performing assays on the test sample and for interpreting the results; reaction reagents; reaction media where reaction is performed; base material to provide the site of assay; and so on. Further, the kit may also comprise reference samples for use as standards for comparison, or for use in preparing calibration curves, detectors, and the like, if desired.

Hereinbelow, the present invention will be described with reference to specific Examples. However, the present invention is not limited to these Examples.

### [EXAMPLE 1] Cloning and Identification ofTannase Gene

### (1) Bacterial Strains and Vector Used

Tannase-producing *Staphylococcus (S.) lugdunensis* 315-1 isolated from feces of colon cancer patients was used as a tannase gene donor strain. As a host for cloning, *Escherichia (E.) coli* DH5α [supE44, ΔlacU169, (Δ80lacZΔM15), recA1, endA1, hsdR17 (rₖ⁻,mₖ⁺), thi-1, gyrA96, relA1] was used. As a vector, pUC18 was used.

### (2) Media Used

For culturing *S. lugdunensis,* Brain Heart Infusion medium (BHI: Difco) was used. As an agar medium, Brain Heart Infusion Agar medium (Oxoid) was used. For growth-culturing *E. coli,* Luria-Bertani (LB) medium [Bacto Tryptone (Difco) 10 g, Bacto Yeast Extract (Difco) 5 g, NaCl 10 g, purified water 1,000 mL (pH 7.2)] was used. The agar medium was 1.5 % agar (Bacto Agar: Difco)-added liquid medium. For transformation of E. *coli,* SOB liquid medium [Bacto Tryptone 4 g, Bacto Yeast Extract 1 g, NaCl 116 mg, KCl 37 mg, purified water 200 mL (pH 7.0) to which 2 M Mg solution (1 M MgCl₂ and 1 M MgSO₄) 2 mL was added] and SOC liquid medium (SOB medium 200 mL to which 2 M Glucose solution 1 mL was added) were used. For primary selection (screening) of transformant clones, LB agar medium containing 50 µg/mL ampicillin (Wako Pure Chemical Industries, Ltd), 50µg/mL 5-bromo-4-chloro-1H-indol-3-yl β-D-galactopyranoside (X-gal), 200 µg/mL isopropyl β-D(-)-thiogalactopyranoside (IPTG) was used. For screening the tannase activity of transformant clones, tannin-treated BHI agar medium was used which was prepared by treating the surface of BHI plate agar medium with 2 % (w/v) tannic acid (Kanto Chemical Co., Inc.) phosphate buffer (PBS: 0.14 M NaCl, 2.7 mM KCl, 8.1 mM Na₂HPO₄·12H₂O, 1.5 mM KH₂PO₄) sterilized with a membrane filter (0.45 µm: ADVANTEC).

### (3) DNA Extraction and Purification

### (3-1) Extraction of S. lugdunensis Chromosomal DNA

One mL of the culture broth of *S. lugdunensis* shaking-cultured at 37°C overnight in BHI liquid medium was transferred into a microcentrifuge tube and centrifuged (15,000 rpm, 1 minute) to harvest cells. After removal of the supernatant, cell pellet was suspended in 200 µL of cell resuspension solution [50 mM Tris (pH 7.5), 10 mM EDTA 2Na, 100 µg/mL RNase A]. To the resultant cell suspension, 100 µg/mL achromopeptidase-containing cell resuspension solution (100 µL) was added, and lysis treatment was performed at 50°C for 30 minutes. 2% Sodium lauryl sulfate (SDS) (300 µL) was added for cell lysis. Then, 600 µL of phenol-chloroform solution [equilibrated phenol 50 mL, chloroform 48 mL, isoamyl alcohol 2 mL, 0.1 M Tris-HCl (pH 8.0) 10 mL] was added thereto and mixed sufficiently. The mixture was centrifuged (15,000 rpm, 5 minutes), and the aqueous layer of the supernatant was collected. Two volumes of ethanol was added thereto, which was then centrifuged (15,000 rpm, 5 minutes) to collect chromosomal DNA as a precipitate. After removal of the supernatant, an appropriate amount of 70% ethanol was added to the precipitate. After re-centrifugation, the supernatant was removed. After removal of ethanol residue by vacuum centrifugation, the precipitate was dissolved in sterilized water to thereby obtain a purified chromosomal DNA solution.

### (3-2) Extraction of Plasmid DNA

Wizard Plus Minipreps Purification System (Promega) was used for extraction of plasmid DNA. Briefly, cells were inoculated into LB liquid medium and shaking-cultured at 37°C overnight. Two mL of the culture broth was centrifuged (15,000 rpm, 1 minute) to harvest cells. After removal of the supernatant, the precipitate was suspended in 300 µL of cell resuspension solution. Cell lysis solution [0.2 M NaOH, 1% SDS] (300 µL) was added thereto for cell lysis. Neutralization solution [1.32 M CH₃COOK (pH 4.8)] (300 µL) was added thereto and mixed sufficiently. After centrifugation (15,000 rpm, 5 minutes), the supernatant was collected. Wizard Minipreps DNA Purificaton Resin (1 mL) was added to the collected supernatant and mixed sufficiently to thereby allow DNA to be adsorbed on the resin. The resultant resin-mixed liquid was passed through Wizard Minicolumn and then washed with column wash solution [80 mM CH₃COOK, 8.3 mM Tris-HCl (pH 7.5), 40 µM EDTA-2Na, 55 % ethanol] (2 mL). Subsequently, plasmid DNA was eluted with water to thereby obtain a purified plasmid DNA.

### (4) Digestion with Restriction Enzyme and DNA Ligation Reaction

### (4-1) Digestion Reaction with Restriction Enzyme

DNA (1-3 µg) was dissolved in 50 µL, and about 10 units of a restriction enzyme was added thereto and reacted for 2 hours. The reaction solution and the reaction temperature were according to the optimum conditions for the restriction enzyme.

### (4-2) DNA Ligation Reaction

One to three µg of DNA fragment, 1000-2000 units of T4 DNA ligase (Takara Shuzo Co.) and 2 µL of T4 DNA Ligation buffer [660 mM Tris-HCl (pH 7.6), 66 mM MgCl₂, 100 mM DTT, 1 mM ATP] were mixed, and the total volume was adjusted to 20 µL. The resultant mixture was reacted at 16°C for 16 hours.

### (5) Transformation into E. coli

The transformation into E. *coli* was performed according to the method of Hanahan.

### (5-1) Preparation of Competent Cells

*E. coli* cells grown in LB agar medium were inoculated into 10 mL of SOB liquid medium and shaking-cultured at 37°C. When O.D. _{at 600 nm} reached approx. 0.5, cells were immediately transferred into microcentrifuge tubes by 1 mL and left in ice for 5 minutes. Then, cells were harvested by centrifugation (1,5000 rpm, 1 minute) and the supernatant was discarded as much as possible. Ice-cooled CPG buffer [100 mM KCl, 50 mM CaCl₂·2H₂O, 10 % Glycerol, 10 mM CH₃COOK(pH 7.5)] (500 µL) was added to the cells, which were suspended therein with a mixer. The resultant cell suspension was left in ice for 30 minutes and then centrifuged (1,5000 rpm, 1 minute) and the supernatant was discarded as much as possible. Ice-cooled CPG buffer (100 µL) was added to the cells, which were suspended sufficiently with a mixer to thereby obtain a competent cell suspension.

### (5-2) Method of Transformation

The competent cell suspension (100 µL) and the DNA solution (1-10 µL) were put in a sterilized microcentrifuge tube and mixed gently. The tube was left in ice for 30 minutes and then heated at 42°C for 1 minute. Then, the tube was immediately returned into ice and left for 2-5 minutes. Subsequently, the contents of the tube were added to 1 mL of SOC liquid medium and shaking-cultured for 60 minutes. The resultant culture broth was smeared by 0.15 mL on LB agar medium containing a selective drug, and cultured overnight at 37°C. The resulting colonies were obtained as transformant clones.

### (6) Determination of DNA Nucleotide Sequence

### (6-1) Amplification and Purification of DNA

PCR was performed in a thermal cycler GeneAmp PCR system 9700 using pUC18 into which a DNA to be sequenced is inserted as a template, pUC-F (SEQ ID NO: 3) and pUC-R (SEQ ID NO: 4) as primers and LA Taq^{™} (Takara), to thereby amplify the inserted DNA fragment. Briefly, after DNA denaturation by heating at 95° for 2 minutes; 5 cycles of denaturation at 95°C for 30 seconds, annealing at 55°C for 30 seconds and extension at 72°C for 4 minutes; then 25 cycles of denaturation at 94°C for 20 seconds, annealing at 55°C for 20 seconds and extension at 72°C for 4 minutes; and final extension at 72°C for 10 minutes were performed. Purification of the DNA was performed with QIAquick PCR Purification Kit (QIAGEN).

pUC-F (SEQ ID NO: 3): gatgtgctgcaaggcgaattgt

pUC-R (SEQ ID NO: 4): gctcgtatgttgtgtggaattgtg

### (6-2) Preparation of Samples for Nucleotide Sequence Determination

For determination of nucleotide sequences, BigDye Terminator Cycle Sequencing FS Ready Reaction Kit (Applied Biosystems) using AmpliTaq DNA polymerase was used. PCR was performed according to the manual attached to the Kit using approx. 30 ng of the DNA (the PCR product) obtained by PCR described in (1). The reaction solution was purified with Cetri-Sep spin column (Applied Biosystems) and then vacuum spin-dried. Subsequently, the resultant DNA was dissolved in 4 µL of a loading solution [deionized formamide 5 mL, blue dextran solution (50 mg/mL blue dextran, 25 mM EDTA) 1 mL] when it was subjected to polyacrylamide gel electrophoresis, or dissolved in 20 µL of Template Suppression Reagent (TSR: ABI PRISM) when it was subjected to capillary electrophoresis. Samples to be sequenced were heated at 95°C for 2 minutes, then immediately ice-cooled, and set in an automated sequencer ABI PRISM^{™} 377 DNA Sequencer (Applied Biosystems) or ABI PRISM^{™} 310 DNA Sequencer (Applied Biosystems) for determination of the DNA nucleotide sequences thereof

### (6-3) Analysis of Nucleotide Sequences

Data analysis of the resultant DNA nucleotide sequences was performed using DNA Sequence Analysis Software^{™}(ABI PRISM), AutoAssembler^{™} Software Version 2.1 (ABI PRISM) and DNA analysis program GENETYX-MAC Ver.11 (Software Development).

### (7) Results of Cloning of Tannase Gene

Chromosomal DNA (2 µg) extracted from *S*. *lugdunensis* 315-1 strain by the SDS-phenol method was partially digested with a restriction enzyme HindIII. To this digest, 1 µg of pUC18 completely digested with HindIII was added, followed by ethanol precipitation. These mixed DNAs were ligated and transformed into *E. coli* DH5α strain. The transformants were smeared on LB agar medium containing 50 µg/mL ampicillin, 50 µg/mL X-gal and 200 µg/mL IPTG for primary selection. About 3,000 white colonies grown were replicated to tannic acid medium and cultured at 37°C for 72 hours under anaerobic conditions. Three clones (Tan18, Tan24 and Tan37) around which a clear zone formed by degradation of tannic acid was observed were isolated. The results of PCR using primers pUC-F and pUC-R located near to the HindIII restriction site of pUC18 and the results of analysis of the digestion with the restriction enzyme revealed that all these three clone have an approx. 7 kb DNA fragment inserted into the HindIII restriction site of pUC18. The analysis of digestion with the restriction enzyme showed different digestion patterns in the DNA inserted into the vector, but the clones had a common DNA fragment other than the vector. The DNA nucleotide sequence of pTZtan18 carried by the transformant clone Tan18 was determined.

### (8) Determination of the DNA Nucleotide Sequence of Tannase Gene

The inserted DNA fragment of the recombinant vector pTZtan18 obtained was amplified with pUC-F and pUC-R primers to thereby obtain a template for determining the nucleotide sequence of the tannase gene. The DNA nucleotide sequence was determined by primer walking by serially designing primers with DNA sequencer 310. The resultant DNA nucleotide sequence was re-confirmed by analysis using DNA sequencer 377. A physical map for the determined DNA nucleotide sequence (SEQ ID NO: 5) is shown in Fig. 1. The DNA fragment comprising the cloned tannase gene from *S*. *lugdunensis* consists of 6,944 bp with G+C contents of 34.7%. From the analysis of the gene, three open reading frames (ORFs) capable of coding proteins have been detected. ORF1 was capable of encoding a 69,190 kd protein consisting of 635 amino acids (aa) starting from the initiation codon ATG at 653 nucleotide (nt) and ending at the termination codon at 2,557 nt. ORF2 was capable of encoding a 54,987 kd protein consisting of 479 aa starting from the initiation codon ATG at 2,581 nt and ending at the termination codon at 4,017 nt. ORF3 was capable of encoding a 67,971 kd protein consisting of 613 aa starting from the initiation codon ATG at 6,034 nt and ending at the termination codon at 4,196 nt.

### (9) Identification of Tannase Gene

### (9-1) Preparation of DNA Fragment Deletion Vector

Using the pTZtan18 of which the DNA nucleotide sequence was determined, ORF3 was deleted. Restriction enzyme EcoRI restriction site was located on ORF3 and pUC18 alone. Then, pTZtan18 was digested with EcoRI, followed by self-ligation and transformation into *E*. *coli* DH5α. As a result of selection using 50 µg/mL ABPC-containing LB agar medium, a plurality of clones were isolated which carried a deletion vector pTZtan18EcRI with an approx. 4 kb insert. When those clones carrying pTZtan18EcRI were cultured for 3 days or more in a tannic acid medium, no clear zone was observed (Fig. 1).

### (9-2) Subcloning ofORF2 and ORF3

Using pTZtan18 as a template, DNA fragments comprising ORF2 and ORF3 were amplified separately by PCR. For amplifying the DNA fragment comprising ORF2, primers Tan-ORF2-F(Bam) (SEQ ID NO: 6) and Tan-ORF2-R(Sal) (SEQ ID NO: 7) were used. For amplifying the DNA fragment comprising ORF3, primers Tan-ORF3-F(Sal) (SEQ ID NO: 8) and Tan-ORF3-R(Bam) (SEQ ID NO: 9) were used. At each of the 5' ends of these primers, BamHI or SalI restriction recognition sequences was added in advance. Each of the amplified DNA fragments was digested with restriction enzymes BamHI and SalI, integrated between the BamHI and SalI restriction sites of pUC18, and transformed into E. coli DH5α. As a result of transformant selection using 50 µg/mL ABPC-containing LB agar medium, clones carrying pTZtanl8-ORF2P (into which an approx. 2.1 kb DNA fragment comprising ORF2 was inserted) and clones carrying pTZtan18-ORF3P (into which an approx. 2.6 kb DNA fragment comprising ORF3 was inserted) were isolated. When these clones were cultured in a tannic acid medium for 3 days or more, a definite clear zone was observed in clones carrying pTZtan18-ORF3P, while no clear zone degrading tannic acid was observed around colonies in clones carrying pTZtanl8-ORF2P (Fig. 1). Therefore, it has become clear that the gene encoding tannase is ORF3.
Tan-ORF2-F(Bam) (SEQ ID NO: 6): gaaagaatccaatatttgcggaaggtatg
Tan-ORF2-R(Sal) (SEQ ID NO: 7): gaaagtcgacttcaaaacagtttggga
Tan-ORF3-F(Sal) (SEQ ID NO: 8): gaaagtcgactggtatcgcaaagttattg
Tan-ORF3-R(Bam) (SEQ ID NO: 9): gaaaggatccacctcatgataatcattct

### [EXAMPLE 2] Preparation of Tannase Protein Expression Vector

### (1) Bacterial Strains and Vector Used

As hosts, E. *coli* JM109 [recA1, endA1, gyrA96, thi, hsdR17, supE44, relA1, Δ(lac-proAB)/F'(traD36, proAB, lacI^{q}, lacZΔM15))] and BL21 [F-, ompT, hsdSb(r_{B}⁻ m_{B}), gal(λ, cI857, ind1, Sam7, nin5, lacUV5-T7genel)] were used. As a vector, pGEX-4T-1 (Amersham Pharmacia Biotech) [ABPC^{r}, Ptac, laqI^{q}, gst gene] was used.

### (2) Digestion with Restriction Enzyme and DNA Ligation Reaction

Restriction enzyme treatment and DNA ligation reaction were performed in the same manner as described in Example 1.

### (3) Transformation of E. coli

Transformation into E. coli was performed by the method of Hanahan, in the same manner as described in Example 1.

### (4) Preparation of Expression Vector

### (4-1) PCR

Forward primer ORF3-F(BamHI) (SEQ ID NO: 10) was designed so that it has a BamHI recognition sequence located at 5' side of the ATG initiation codon of ORF3; and reverse primer ORF3-R(XhoI) (SEQ ID NO: 11) was designed so that it has an XhoI recognition sequence located at 3' side of the termination codon TAG of ORF3. Synthesis of these primers were performed by SIGMA.

The DNA fragment of ORF3 was amplified by PCR using pTZtan18 as a template and ORF3-F(Bam) and ORF3-R(Xho) as primers.
ORF3-F(BamHI) (SEQ ID NO: 10): gaaaggatccatgaaaaagactttcatatcactctt
ORF3-R(XhoI) (SEQ ID NO: 11): ggaaaactcgagctattttttattaatactttctaccc

The PCR was performed using KOD DNA Polymerase (Toyobo). Five µL of 2 mM dNTPs (Toyobo), 2 µL of 25 mM MgCl₂, 5 µL of 10 x Buffer #1 (Toyobo) [1.2M Tris-HCl (pH 8.0), 100 mM KCl, 60 mM (NH₄)₂SO₄, 1% Triton X-100, 100 µg/mL BSA], 25 pmol of each primer, 2 µL of template DNA, 2.5 units of KOD DNA Polymerase, and ultrapure water to make the total volume 50 µL were mixed to prepare a reaction solution. The reaction was performed in a thermal cycler GeneAmp PCRSystem 9700 under the following conditions. Briefly, after DNA denaturation by heating at 95° for 2 minutes; 5 cycles of denaturation at 95°C for 30 seconds, annealing at 55°C for 30 seconds and extension at 74°C for 2 minutes; then 20 cycles of denaturation at 94°C for 20 seconds, annealing at 60°C for 20 seconds and extension at 74°C for 2 minutes; and final extension at 74°C for 5 minutes were performed. The PCR product was purified using QIAquick PCR Purification Kit (QIAGEN).

### (4-2) Insertion into Expression Vector

The purified DNA fragment and the expression vector pGEX-4T-1 were digested with restriction enzymes BamHI and XhoI, and mixed. After ligation reaction with T4 DNA ligase, the resultant DNA was transformed into *E. coli* JM109 strain. Transformants were selected in 50 µg/mL ABPC-containing LB agar medium. The ABPC-resistant transformant clones grown were cultured in a tannic acid medium for 3 days or more. When the plasmids in ABPC-resistant transformant clones around which a clear zone had been observed were examined, it was found that an approx. 1.8 kb DNA fragment was inserted in every plasmid. Of these clones, 4 clones (pGEXtan8, pGEXtan35, pGEXtan65 and pGEXtan13) were analyzed further on the digestion patterns of plasmids using restriction enzymes EcoRI, BamHI and XhoI. As a result, an identical band was confirmed in all the 4 plasmids.

### (5) Confirmation of the Nucleotide Sequence of the Inserted DNA

With respect to pGEXtan8 and pGEXtan65 of the above 4 plasmids, the inserted DNA fragments were amplified separately by PCR using these plasmids as templates, GST-1(+) (SEQ ID NO: 12) and GST-2(-) (SEQ ID NO: 13) as primers and KOD DNA Polymerase. Then, the nucleotide sequences of the inserted DNA fragments were confirmed. The determination and analysis of the nucleotide sequences were performed in the same manner as described in Example 1.

In these two types of plasmids, the nucleotide sequences of the inserted DNA were completely identical; and the tannase gene was inserted in frame with glutathione S-transferase. It was confirmed that a BamHI recognition sequence is located at 5' side of the initiation codon ATG of tannase gene and that an XhoI recognition sequence is located at 3' side of the termination codon TAG
GST-1(+) (SEQ ID NO: 12): gggctggcaagccacgtttggtg
GST-2(-) (SEQ ID NO: 13): ccgggagctgcatgtgtcagagg

### [EXAMPLE 3] Detection of Tannase Gene by PCR from Various Standard Strains and Clinically Isolated Strains

### (1) Strains Used

As standard strains, *Staphylococcus lugdunensis* ATCC43809, *Staphylococcus aureus* ATCC25923, *Staphylococcus epidermidis* ATCC14990, *Staphylococcus hominis* JCM2419, *Staphylococcus schleiferi* ATCC43808, *Escherichia coli* ATCC11775, *Bacillus subtilis* ATCC6633, *Enterococcus faecalis* ATCC19433, *Lactobacillus plantarum* TOA1088 and *Streptococcus gallolyticus* ACM3611 were used.

As tannase positive, clinically isolated strains from human feces, *Staphylococcus lugdunensis* 315-1, *Lactobacillus plantarum* 67, *Lactobacillus pentosus* St067 and *Streptococcus bovis* 303 were used.

### (2) Method of Preparation of PCR Samples from Standard Strains and Clinically Isolated Strains

Various strains and clinically isolated strains were grown on agar plate growth medium. One colony was scraped off with a sterilized toothpick from each strain and suspended in 100 µl of sterilized water. This suspension was used as a DNA sample solution for PCR.

### (3) Method of Amplification of Tannase Gene by PCR

To 1µl of the DNA sample for PCR, 50 pmol of each primer and 12.5 µl of PCR Master Mix (Promega) were added. Sterilized purified water was added thereto to make the total volume 25 µl. As primers, Tan-F-F6 (SEQ ID NO: 14) and Tan-F-R7 (SEQ ID NO: 15) were used. Tannase gene was amplified in a thermal cycler GeneAmp PCR system 9700 (Applied Biosystems) under the following conditions. Briefly, after disruption of cells by heating at 95° for 3 minutes; 5 cycles of DNA denaturation at 5°C for 30 seconds, annealing at 50°C for 30 seconds and extension at 72°C for 30 seconds; then 25 cycles of DNA denaturation at 94°C for 20 seconds, annealing at 50°C for 20 seconds and extension at 72°C for 30 seconds; and final extension at 72°C for 5 minutes were performed. The amplified product was electrophoresed in 2.5% agarose X gel (Nippon Gene), and the presence or absence of tannase gene (223 bp) was examined.
Tan-F-F6 (SEQ ID NO: 14): cgtggtgccctattacaagt
Tan-F-R7 (SEQ ID NO: 15): tgagccttcagtatattgacca

### (4) Results of Detection of Tannase Gene by PCR from Standard Strains and Clinically Isolated Strains

Detection of tannase gene was performed by PCR on the total 14 strains of standard strains and clinically isolated strains. As a result, tannase gene was detected in only two *Staphylococcus lugdunensis* strains. From these results, it has become clear that the tannase gene of the present invention is a gene specific to *Staphylococcus lugdunensis.*

### [EXAMPLE 4] Detection of Tannase Gene from Human Feces by PCR

### (1) Method of Preparation of PCR Samples from Human Feces

A cotton swab inserted into the anus of a subject was placed in *Streptococcus* selection supplement-containing Brain Heart Infusion (BHI) liquid medium (2 mL) and cultured overnight anaerobically at 37°C. One hundred µl of the resultant culture broth was mixed with 500 µl of sterilized water and centrifuged at 1500 rpm for 1 minute to precipitate the cultured cells. After removal of the supernatant, 100 µl of sterilized water was added to prepare a cell suspension, which was used as a DNA sample for PCR

### (2) Method of Amplification of Tannase Gene by PCR

To 3 µl of the DNA sample for PCR, 50 pmol of each primer and 12.5 µl of PCR Master Mix (Promega) were added. Sterilized purified water was added thereto to make the total volume 20 µl. As primers, Tan-F-F6 (SEQ ID NO: 14) and Tan-F-R7 (SEQ ID NO: 15) were used. Tannase gene was amplified in a thermal cycler GeneAmp PCR system 9700 (Applied Biosystems) under the following conditions. Briefly, after disruption of cells by heating at 95° for 3 minutes; 5 cycles of DNA denaturation at 95°C for 30 seconds, annealing at 50°C for 30 seconds and extension at 72°C for 30 seconds; then 25 cycles of DNA denaturation at 94°C for 20 seconds, annealing at 50°C for 20 seconds and extension at 72°C for 30 seconds; and final extension at 72°C for 5 minutes were performed. The amplified product was electrophoresed in 2.5% agarose X gel (Nippon Gene), and the presence or absence of tannase gene (223 bp) was examined.

### (3) Results of Detection of Tannase Gene from Human Feces by PCR

Detection of tannase gene was performed by PCR on the bacteria obtained from human fecal samples from 4 subjects. At the same time, the bacteria were cultured in tannic acid-over layered Brain Heart Infusion Agar medium (Oxoid). Clear zones indicating tannase production were confirmed, and tannase-producing bacteria were isolated and identified.

As a result, tannase gene was detected from the fecal sample of one subject by PCR. Besides, clear zones indicating tannase production were confirmed by the culture method, and *Staphylococcus lugdunensis* was isolated and identified. With respect to the bacteria obtained from the remaining 3 subjects, tannase gene was not detected by PCR; tannase production was negative in the culture method; and *Staphylococcus lugdunensis* was not isolated.

From these results, it was found that tannase-producing bacteria in human feces can be detected by detecting tannase gene by PCR

### [EXAMPLE 5] Production of GST-Fused Tannase Protein by E. coli

### (1) Expression Vector Used and Expression of GST-Fused Tannase Protein

*E. coli* BL21 strain in which the gene recombinant tannase protein expression vector (pGEXtan8) prepared in Example 2 had been integrated was used. Briefly, *E. coli* was shaking-cultured overnight at 37°C in 50 µg/mL ampicillin (Wako Pure Chemical Industries, Ltd)-containing LB medium (1 L). Then, isopropyl β-D(-)-thiogalactopyranoside (Sigma) was added thereto to give a concentration of 0.5 mM, followed by shaking culture for 4 hours.
Grown cells were transferred into a centrifuge tube and centrifuged (8,000 rpm, 5 minutes) to harvest cells.

### (2) Purification of GST-Fused Tannase Protein

To the harvested *E. coli* cells, 1% Triton X-100 (Wako Pure Chemical)-containing phosphate buffer (PBS: 0.14 M NaCl, 2.7 mM KCl, 8.1 mM Na₂HPO₄·12H₂O, 1.5 mM KH₂PO₄) (15 ml) was added to suspend the cells. Then, the cell suspension was frozen overnight at -80°C. After thawing, cells were disrupted with an ultrasonic homogenizer for 3 minutes and centrifuged (10,000 rpm, 10 minutes) to collect the supernatant. The supernatant was fed to Glutathione Sepharose 4B column (gel volume: 2 mL) (Amersham Biosciences) and washed with 20 mL of phosphate buffer. Subsequently, 10 mL of elution buffer (10 mM Glutathione-containing 50 mM Tris-HCl buffer, pH 8.0) was fed to the column to thereby elute the bound GST-fused tannase protein.

### (3) Electrophoretic Analysis of GST-Fused Tannase Protein

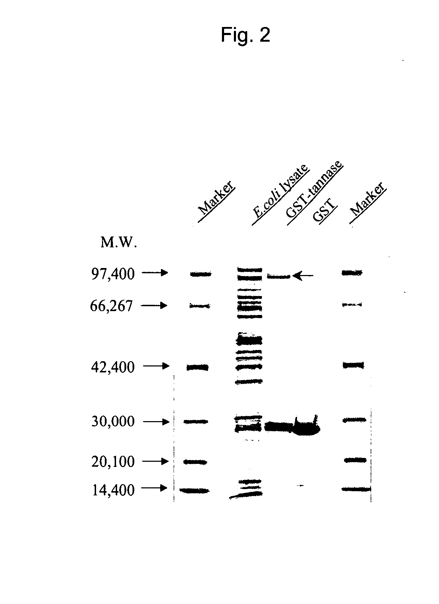

The resultant GST-fused tannase protein was analyzed by SDS polyacrylamide gel (PAG mini "Daiichi" 10/20; Daiichi Pure Chemicals). The results of electrophoresis of E. *coli* lysate (lysate before purification), GST-tannase (purified GST-fused tannase) and GST alone are shown in Fig. 2 together with molecular weight markers. The deduced molecular weight of GST-fused tannase protein predicted from the amino acid sequence based on the nucleotide sequence of the tannase gene is approx. 94,000. A protein band (marked with an arrow) which is believed to correspond to this molecular weight was confirmed.

### (4) Confirmation of Tannase Activity of GST-Fused Tannase Protein

Five µL of the resultant GST-fused tannase protein (100 µg/mL) was dripped to tannic acid-treated BHI agar medium and cultured anaerobically at 37°C for 24 hours. As a result, clear zones generated by degradation of tannic acid were observed at the dripping sites. From this result, it was confirmed that the prepared GST-fused tannase protein has a tannase activity.

### [EXAMPLE 6] Preparation of Anti-Tannase Monoclonal Antibody-Producing Hybridomas

### (1) Mouse Immunization and Cell Fusion

Balb/c mice were immunized with the GST-fused tannase protein prepared in Example 5 four times at intervals of 2 weeks (10 µg/time). Three days after the final immunization, spleen cells were collected from the mice and mixed with mouse myeloma cells (P3U1) (number of spleen cells: number of myeloma cells = 3:1). The cells were fused using polyethylene glycol 1500 (Roche). Then, HAT medium (Gibco) was added thereto, and the resultant mixture was dispensed into five 96-well plates and cultured at 37°C. Ten days thereafter, those wells in which cell colonies were appearing were selected. The culture supernatant of each well was screened for anti-tannase antibody activity.

### (2) Screening of Antibody Activity

Antibody activity was screened by Western blotting. Briefly, GST-fused tannase protein was electrophoresed on SDS polyacrylamide gel (concentration: 12.5%) and then transferred onto Immobilon-P Transfer Membrane (Millipore) to prepare an antigen-transferred membrane. This membrane was cut into strips and placed in a reaction tray A culture supernatant to be screened was added thereto and reacted at room temperature for 1 hour. Then, the antigen-transferred membrane was washed, and HRP-labeled anti-mouse IgG antibody (Zymed) was added thereto and reacted at room temperature for 1 hour. Subsequently, the antigen-transferred membrane was washed and then allowed to develop color by addition of hydrogen peroxide-containing 4-chloro-1-naphtol (Sigma) color developing solution. When color development was observed in the band of GST-fused tannase protein with a molecular weight of 94,000, the culture supernatant was judged positive.

### (3) Cloning

Cells in those wells which were found positive in antibody activity were transferred into 24-well plates. HAT medium was added thereto, and cells were cultured at 37°C for 7 days. The culture supernatant of each well was screened for antibody activity by Western blotting. Then, cells in those wells which were found positive in antibody activity were cloned by the limiting dilution method. Briefly, a series of dilutions were prepared with HAT medium to give cell concentrations of 15 cells/mL, 5 cells/mL and 1.5 cells/mL. For each dilution, 0.2 mL was dispensed into a 96-well plate and cultured at 37°C for 10 days. Those wells in which a single cell colony was appearing in one well were selected, and the culture supernatants of those wells were screened for antibody activity by Western blotting. Two cells in which antibody activity had been observed were selected and designated clone "2B5" and clone "2F6", respectively. (Hereinbelow, the above designations of clones are used for both cell and antibody.)

### (4) Confirmation of Specificity of Antibodies

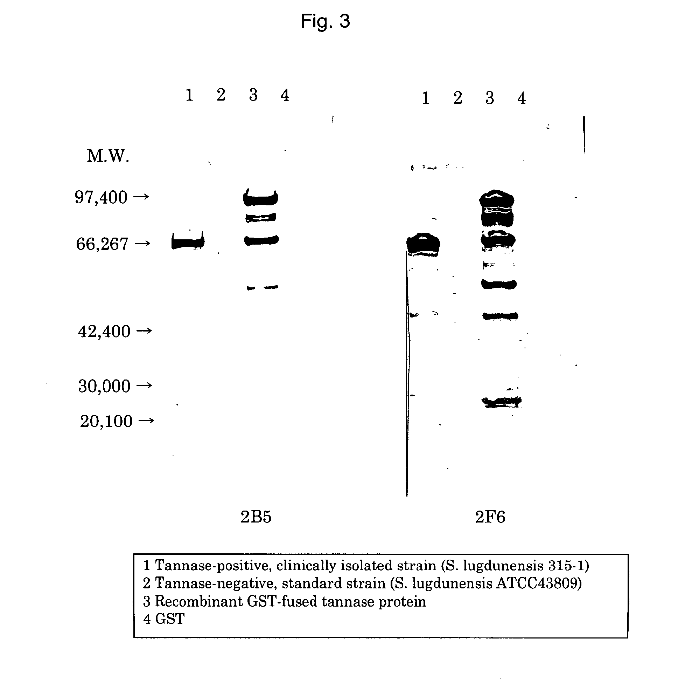

The specificity of antibody was confirmed on the culture supernatants of clones 2B5 and 2F6 by Western blotting. The results are shown in Fig. 3. In lane 1, a cell extract protein from tannase-positive, clinically isolated strain (*S. lugdunensis* 315-1) was transferred; in lane 2, a cell extract protein from tannase-negative, standard strain (*S. lugdunensis* ATCC43809) was transferred; in lane 3, the gene recombinant GST-fused tannase protein used in immunizing mice was transferred; and in lane 4, GST protein alone was transferred. Both clones 2B5 and 2F6 only reacted with a tannase protein having a deduced molecular weight of 68,000 in the tannase-positive, clinically isolated strain in lane 1, but none of them reacted with the tannase-negative, standard strain in lane 2. Further, in the GST-fused tannase protein in lane 3, both clones reacted strongly with a tannase protein having a deduced molecular weight of 68,000 and the GST-fused tannase protein having a deduced molecular weight of 94,000; and they also reacted with several proteins having a rather small molecular weight (which are believed to be tannase degradation products). None of the clones reacted with the GST protein in lane 4. From these results, it was confirmed that the antibodies produced by clones 2B5 and 2F6 are monoclonal antibodies specific to *S. lugdunensis*-derived tannase.

### [EXAMPLE 7] Measurement of Tannase by EIA Method

### (1) Purification ofAnti-Tannase Monoclonal Antibodies

Anti-tannase monoclonal antibody-producing hybridomas 2B5 and 2F6 prepared in Example 6 were cultured in serum-free medium S-Clone (Sankyo Junyaku Co.) at a concentration of 10⁵ cells/mL. Approximately 500 µL of each culture supernatant was collected and fed to Protein G Sepharose 4B column (gel volume: 5 mL) (Amersham Biosciences) to allow IgG to be adsorbed onto the column. Then, the column was washed with 50 mL of phosphate buffer (PBS: 0.14 M NaCl, 2.7 mM KCl, 8.1 mM Na₂HPO₄·12H₂O, 1.5 mM KH₂PO₄). Subsequently, 10 mL of elution buffer (0.1 M Glycine-HCl buffer, pH2.5) was fed to the column to elute the bound IgG. Immediately thereafter, 1/10 volumes of 1 M Tris-HCl buffer (pH 9.0) was added thereto for neutralization.

### (2) EIA Method

Purified 2B5 antibody was mixed with phosphate buffer to give a concentration of 10 µg/mL, added to a 96-well microtiter plate at 100 µL/well, and left overnight at 20°C to thereby immobilize IgG on the surface of the well. Then, the well was washed with purified water, and a test sample was added thereto at 100 µL/well and reacted at 37°C for 1 hour. After the well was washed with a washing solution (0.05 % Tween 20, 0.15M NaCl, 0.01M Tris HCl buffer, pH7.5), HRP-labeled 2F6 antibody adjusted with 10% rabbit serum to give a concentration of 1 µg/mL was added thereto at 100 µL/well and reacted at 37°C for 1 hour. After the well was washed with the washing solution, TMBZ (3,3',5,5'-tetramethylbenzidine; Sigma) as a substrate solution was added thereto at 100 µL/well and reacted at 37°C for 15 minutes. Subsequently, 1 M sulfuric acid was added at 100 µL/well to terminate the reaction, and the absorbance (at 450 nm) was measured with a microplate reader.

### (3) Partial Purification of S. lugdunensis-Derived Tannase

Tannase-positive *S*. *lugdunensis*-315-1 strain isolated from feces of a colon cancer patient was cultured in tannic acid-treated BHI agar medium (Difco) (100 plates 90 mm in diameter) at 37°C for 5 days under anaerobic conditions. After the culture, phosphate buffer (PBS: 0.14 M NaCl, 2.7 mM KCI, 8.1 mM Na₂HPO₄·12H₂O, 1.5 mM KH₂PO₄) was added at 5 mL/plate, and cells grown on the surface of the medium were collected and centrifuged (7,000 rpm, 10 minutes, 4°C) to harvest cells. The resultant cells were suspended in 10 mL of phosphate buffer. To this suspension, 100 µL of lysostaphin (Sigma) adjusted with phosphate buffer to give a concentration of 10 mg/mL was added and reacted 37°C for 30 minutes under aerobic conditions. Further, the cell suspension was sonicated and centrifuged (7,000 rpm, 10 minutes, 4°C). The supernatant was collected as a crude enzyme solution.

Ten mL of this crude enzyme solution was adsorbed onto a column (2.6 x 20 cm) packed with DEAE-Sepharose Fast Flow (Amersham Biosciences). Non-adsorbed substances were eluted with 300 mL of 0.05 M Tris-HCl (pH 7.0) at a flow rate of 5 mL/min. Subsequently, the protein was eluted with a concentration gradient of 0 M to 0.5 M NaCl. The eluate was fractionated by 5 mL, and protein concentration and tannase activity were measured.

The measurement of tannase activity was performed by a color development method based on the method of Osawa et al. ("Visual reading method for detection of bacterial tannase", Appl. Environ. Microbiol., 59; 1251-1252, 1993). Briefly, 10 mM methyl gallate dissolved in phosphate buffer and each of the fractions obtained above were mixed at a ratio of 2:1 and reacted at 37°C for 60 minutes under aerobic conditions. After the reaction, an equivalent amount of saturated NaHCO₃ solution was added. The resultant solution was left stationary for 20 minutes at room temperature. Then, the absorbance at 440 nm was measured with a spectrophotometer.

The fractions with tannase activity obtained by ion exchange chromatography using DEAE-Sepharose Fast Flow were collected, centrifuge concentrated to approx. 2 mL with Centricon Plus-20 (Millipore), and gel filtrated in a column (1.6 x 50 cm) packed with Superose 12 (Amersham Biosciences). As an eluent, 0.05 M NaCl-containing 0.05 MTris-HCl (pH 7.0) was used. The eluent was fed at a flow rate of 5 mL/min, and the eluate was fractionated by 2 ml. For each fraction, protein concentration and tannase activity were measured, and those fractions with tannase activity were collected..

In a similar manner, a tannase-negative, standard strain (*S. lugdunensis* ATCC43809) was cultured in tannic acid-untreated BHI agar medium. In the purification process, fractions corresponding to the above-described tannase fractions were collected.

### (4) Results of Tannase Measurement by EIA Method

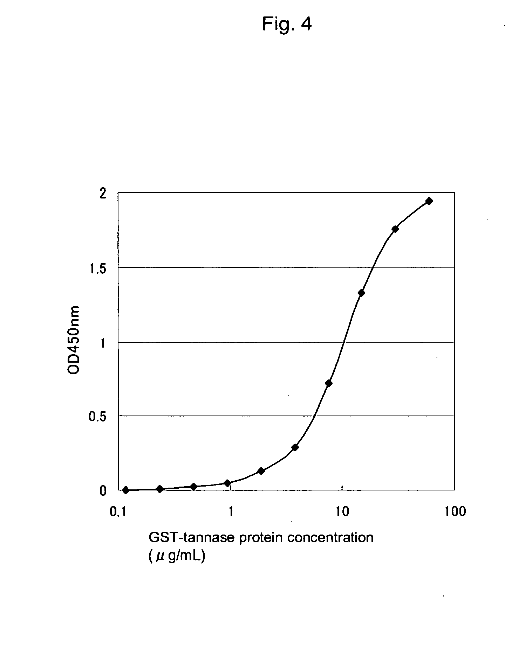

The amount of recombinant GST-fused tannase protein was quantitatively determined with Coomassie Protein Assay Kit (Pierce) using BSA as a standard. Then, the recombinant protein was 2-fold serially diluted from 60 µg/mL to 0.117 µg/mL with phosphate buffer, followed by measurement of absorbance by EIA method. The relation between the concentration of GST-fused tannase protein and EIA absorbance is shown in Fig. 4. A good calibration curve was obtained.

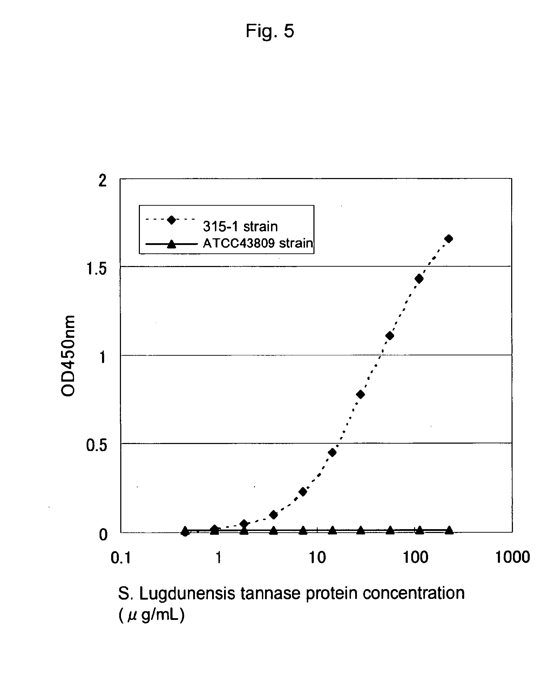

Subsequently, with respect to the tannase protein partially purified from tannase-positive *S*. *lugdunensis* 315-1 strain and the protein corresponding to tannase partially purified from tannase-negative *S. lugdunensis* ATCC43809 strain, the amounts of proteins were quantitatively determined with Coomassie Protein Assay Kit (Pierce) using BSA as a standard. Then, the above two proteins were 2-fold serially diluted from 230 µg/mL to 0.449 µg/mL with phosphate buffer, followed by measurement of absorbance by EIA method. The relations between the concentrations of the tannase protein and the protein corresponding to tannase both partially purified from each strain and EIA absorbances are shown in Fig. 5. When the tannase protein partially purified from tannase-positive *S. lugdunensis* 315-1 strain was measured, a good calibration curve was obtained. On the other hand, the antibodies did not react with the protein corresponding tannase partially purified from tannase-negative *S. lugdunensis* ATCC43809 strain at all. From these results, it has been proved that this EIA method is capable of measuring specifically the tannase produced by *S. lugdunensis* isolated from colon cancer patients.

### INDUSTRIAL APPLICABILITY

By a gene detection method utilizing the nucleotide sequence of the tannase gene of the present invention, highly sensitive detection of tannase-producing bacteria in feces has become possible. Further, by introducing the tannase gene into an appropriate host, it becomes possible to produce the tannase protein in a large quantity, and it is also possible to produce antibodies to the tannase protein. By an immunological method using the antibody, it becomes possible to measure the tannase in feces simply and promptly.

### SEQUENCE LISTING FREE TEXT

SEQ ID NO: 3: synthetic DNA
SEQ ID NO: 4: synthetic DNA
SEQ ID NO: 6: synthetic DNA
SEQ ID NO: 7: synthetic DNA
SEQ ID NO: 8: synthetic DNA
SEQ ID NO: 9: synthetic DNA
SEQ ID NO: 10: synthetic DNA
SEQ ID NO: 11: synthetic DNA
SEQ ID NO: 12: synthetic DNA
SEQ ID NO: 13: synthetic DNA
SEQ ID NO: 14: synthetic DNA
SEQ ID NO: 15: synthetic DNA

## Claims

1. A polynucleotide encoding a protein as defined in the following (a), (b) or (c):
(a) a protein comprising the amino acid sequence as shown in SEQ ID NO: 2;
(b) a protein comprising the amino acid sequence as shown in SEQ ID NO: 2 with one or several amino acids substituted, deleted, inserted or added, and having a tannase activity;
(c) a protein comprising an amino acid sequence showing 90% or more homology to the amino acid sequence as shown in SEQ ID NO: 2, and having a tannase activity.

2. A polynucleotide as defined in the following (a) or (b):
(a) a polynucleotide comprising a DNA comprising the nucleotide sequence as shown in SEQ ID NO: 1;
(b) a polynucleotide which hybridizes to a DNA consisting of a nucleotide sequence complementary to a DNA comprising the nucleotide sequence as shown in SEQ ID NO: 1 under stringent conditions, and which encodes a protein having a tannase activity.

3. A protein as defined in the following (a), (b) or (c):
(a) a protein comprising the amino acid sequence as shown in SEQ ID NO: 2;
(b) a protein comprising the amino acid sequence as shown in SEQ ID NO: 2 with one or several amino acids substituted, deleted, inserted or added, and having a tannase activity;
(c) a protein comprising an amino acid sequence showing 90% or more homology to the amino acid sequence as shown in SEQ ID NO: 2, and having a tannase activity.

4. A vector comprising the polynucleotide according to claim 1 or 2.

5. A host cell comprising the polynucleotide according to claim 1 or 2 or the vector according to claim 4.

6. A method of producing the protein according to claim 3, comprising culturing the host cell according to claim 5 and collecting the resultant protein from the host cell or culture supernatant thereof

7. A polynucleotide for use in detecting *Staphylococcus lugdunensis,* which hybridizes to the polynucleotide according to claim 1 or 2 under stringent conditions and has a strand length of at least 15 nucleotides.

8. An antibody or a fragment thereof which binds to the protein according to claim 3.

9. The antibody or fragment thereof according to claim 8, wherein the antibody is a monoclonal antibody.

10. A monoclonal antibody or a fragment thereof which binds to tannase, wherein said monoclonal antibody is produced by a hybridoma obtained by cell fusion between a myeloma cell and an antibody producing cell from an animal immunized with the protein according to claim 3 or a part thereof as an antigen.

11. A hybridoma which is obtained by cell fusion between a myeloma cell and an antibody producing cell from an animal immunized with the protein according to claim 3 or a part thereof as an antigen, and produces a monoclonal antibody that binds to tannase.

12. A method of producing the monoclonal antibody, comprising culturing the hybridoma according to claim 11 and collecting from the resultant culture a monoclonal antibody that binds to tannase.

13. A method of detecting *Staphylococcus lugdunensis* by contacting the polynucleotide according to claim 7 with a test sample.

14. A method of detecting or diagnosing colon cancer, comprising detecting *Staphylococcus lugdunensis* by contacting the polynucleotide according to claim 7 with a test sample.

15. A method of detecting tannase-producing *Staphylococcus lugdunensis* by contacting the antibody or fragment thereof according to any one of claims 8 to 10 with a test sample.

16. A method of detecting or diagnosing colon cancer, comprising detecting a tannase-producing *Staphylococcus lugdunensis* by contacting the antibody or fragment thereof according to any one of claims 8 to 10 with a test sample.

17. A diagnostic reagent for colon cancer, comprising the polynucleotide according to claim 7.

18. A diagnostic reagent for colon cancer, comprising the antibody or fragment thereof according to any one of claims 8 to 10.

19. A kit for detecting *Staphylococcus lugdunensis* or colon cancer, comprising at least one selected from the group consisting of the polynucleotide according to claim 7 and the antibody or fragment thereof according to any one of claims 8 to 10.
